# EUROPEAN PATENT APPLICATION

(11) **EP 3 683 305 A1**
(43) Date of publication of application: **22.07.2020**
(21) Application number: 18855987.6
(22) Date of filing: 16.09.2018
(51) Int. Cl.: C12N 5/077, C12N 5/0775, A61K 35/51

(54) **METHOD FOR OBTAINING A COMPOSITION CONTAINING A SPECIFIC POPULATION OF UMBILICAL CORD MESENCHYMAL CELLS AND USES THEREOF**

(30) Priority: 16.09.2017 CL 20172357
(71) Applicant: Cells For Cells S.A., Santiago 7550101 (CL)
(72) Inventor: MAROUN, Khoury, Santiago 7550101 (CL); FIGUEROA, Fernando, Santiago 7550101 (CL); ESPINOZA, Francisco, Santiago 7550101 (CL); BARTOLUCCI, Jorge, Santiago 7550101 (CL); CASTRO, Diego, Santiago 7550101 (CL); MONTOYA, Paula, Santiago 7550101 (CL); GONZÁLEZ, Paz, Santiago 7550101 (CL); CUENCA, Jimena, Santiago 7550101 (CL)
(74) Representative: Herrero & Asociados, S.L.
(86) International application number: PCT/CL2018/050087
(87) International publication number: WO 2019/051623

(57) **Abstract**

The present invention discloses a method for obtaining a specific population of umbilical cord mesenchymal cells. A composition containing said population of specific cells is useful for preparing a medicinal product that can be used in cell therapy, such as cardiac ejection flow problems in patients with a reduced ejection fraction. Moreover, said sub-population is useful for preparing a medicinal product that can be used to relieve pain and restore function in cases of joint wear; to treat respiratory problems, such as lung damage, acute lung injuries, such as acute respiratory distress syndrome, and to treat skin lesions, such as chronic wounds and ulcers.

## Description

The present invention provides a method for obtaining a specific population of umbilical cord mesenchymal cells. A composition containing this population of specific cells is useful for preparing a medicament that is used for cell therapies, such as problems with cardiac ejection flow of patients with a decreased ejection fraction. At the same time, this subpopulation is useful for preparing a medication that serves to relieve pain and restore the functionality of joint wear; to treat respiratory problems, such as lung lesions, acute lung lesions, such as acute respiratory distress syndrome and to treat skin lesions, such as chronic wounds and ulcers.

### Field of the invention

The present invention is contained within the field of medicine. In particular, the present invention provides a method for obtaining a specific population of umbilical cord mesenchymal cells, which serve to obtain a composition for the treatment and / or prevention of ejection deficiencies in patients with heart failure; to relieve pain and improve functionality in patients with joint wear problems; to treat respiratory problems and for skin lesions.

### Background of the invention

Human multipotent stromal cells (h-MSCs) are stromal cells that possess specific characteristics such as the presentation of a specific immunophenotype, adhesion and differentiation to different cell lines, as defined by the "International Society for Cell Therapy" [1]. Among other characteristics that these cells possess is the immunomodulation capacity since they can inhibit the response of alloreactive T lymphocytes.

Considering this background is that h-MSCs have been considered for new cell therapies. Cellular therapies, whether autologous or allogeneic, have been widely studied in various diseases and conditions such as Parkinson's disease, amyotrophic lateral sclerosis, multiple sclerosis, radiation-induced intestinal injury, inflammatory bowel disease, liver disease, Duchenne muscular dystrophy and diabetes, which have shown different results [2,3].

A source of h-MSC are cells obtained from the umbilical cord. However, these cells are heterogeneous, express different surface markers and have different differentiation capabilities, for this reason, h-MSCs must be selected. There is currently a consensus in the scientific community to define h-MSC cells as such, which considers identity and purity criteria based on the expression profile of surface markers. Specifically, for a cell to be considered a mesenchymal stem cell, it must express the markers: CD73, CD90 and CD105, while the markers CD45, CD14, CD19 must be absent. The selection of cells with this expression pattern is done in order to prevent any contamination with other types of cells. It can also be selected by the negative expression of CD34, a marker that is used to rule out contamination with hematopoietic precursors. Additionally, HLA-DR, which is one of the major class II histocompatibility complex molecules, a predictive factor of a possible immune response, should be absent [1].

Additionally, in order to be called mesenchymal cells, they must have the potential to be differentiated into adipocytes, chondroblasts and osteoblasts (differentiation in three lineages). Lately it has been discovered that h-MSCs can be obtained from a variety of tissues such as adipose tissue, placental, dental pulp and umbilical cord. What has resulted, therefore, that these cells in addition to having the traditional differentiation of three lineages can also be differentiated into non-traditional lineages to produce cells such as myocytes, endothelial cells, hepatocytes and neuronal cells. It is believed that the mechanisms of self-renewal and differentiation of h-MSCs are conditioned by a set of growth factors, receptors, intracellular signaling molecules and transcription factors. Therefore, these factors can be useful in identifying these factors. take a group of h-MSCs for a specific lineage.

As already mentioned, h-MSCs are multipotent cells with low immunogenic potential that can be isolated from adult tissues including bone marrow, adipose tissue and dental pulp among other sources. The niche of origin represents an essential factor in the evaluation of biological differences between cell types, since the properties of h-MSC can be greatly influenced by microenvironmental changes. Most experimental and clinical studies have used MSC derived from bone marrow, but these cells have disadvantages for clinical application, including an invasive procedure for obtaining cells, and a decrease in the potential for proliferation and differentiation, which is directly related with the age and comorbidity of the donor. In contrast, h-MSC umbilical cord are easily obtainable and in vitro expansion, in addition to having less cell aging and obtaining them has lower ethical concerns.

Our invention relates to obtaining umbilical cord h-MSC for specific cell therapies, treatment and / or prevention of ejection deficiencies in patients with heart failure, it can also be used to relieve pain and improve functionality in patients with problems of wear on the joints, it is also useful for treating respiratory problems, such as lung lesions, and to treat skin lesions, such as chronic wounds and ulcers.

Cell therapy has been evaluated as a treatment for heart failure associated with a decrease in ejection fraction (HFrEF) for more than a decade. Experimental studies report improvements in cardiac function and regeneration of damaged heart tissue through mechanisms that include transdifferentiation, cell fusion and paracrine modulation. Recent reviews suggest that cell therapy is safe and is associated with moderate clinical benefits in survival, left ventricular function and quality of life of patients with HFrEF. Different clinical studies have been conducted in patients with ischemic and non-ischemic heart disease, with different products and cell types and routes of administration. Among these can be found bone marrow mononuclear cells and mesenchymal stem cells (MSC), which can be autologous or allogeneic. These have been administered by intramyocardial, intracoronary injection and exceptionally by intravenous injection.

There are several clinical studies to treat the left ventricular ejection fraction (LVEF) in heart failure. In a clinical study, conducted by Lunde K, et al., Conducted with 100 patients who had a myocardial infarction, autologous bone marrow mononuclear cells (BMC) administered by intracoronary injection were used. As a result, no difference was found between the control group and the treated group with respect to the LVEF [4]. Perin EC. Et al., also conducted a randomized study with BMC cells in patients with heart failure, and did not register any improvement in LVEF among patients treated with BMC compared to the control [5]. Hare J. et al. They conducted a clinical study where allogeneic mesenchymal stem cells from bone marrow were used to treat patients with acute myocardial infarction. The LVEF was measured as one of the results of the study, after 3 months of the beginning of the treatment a non-significant improvement of 3.6% of the LVEF was observed in the treated patients, however this advantage disappears after six months of follow-up. [6].

In another study, patients with chronic heart failure due to ischemic cardiomyopathy were treated with BMC. After a 60-month follow-up, patients treated with BMC showed an improvement in ejection fraction [8]. Using bone marrow cells, Seth et al., Reported that after 3 years of follow-up, 76% of patients belonging to the functional type III class of the "New York Heart Association" had a 5.4% improvement in ejection fraction over control [9].

Therefore, after decades of basic and clinical research, the overall benefit and the best source of cells and route of administration remain unsolved.

Among the problems of joint wear, osteoarthritis (OA) is the most common type of joint disease, a degenerative and progressive disorder that causes chronic pain, disability, worse quality of life and increased mortality. The knee is one of the most commonly affected sites and currently represents a global health problem. On the one hand, despite the slow course of this disease, there is no disease-modifying osteoarthritis drug (DMOAD) and its prevalence is growing due to the aging of the population, affecting 35% of people over 75 years. On the other hand, this disease represents an important economic burden due to the high health costs, including a progressive increase in total knee arthroplasty and premature retirement.

Cellular therapy based on h-MSC can provide a useful biological tool for osteoarthritis, due to its chondrogenic differentiation capabilities and anti-inflammatory properties [10] Preclinical studies have shown positive results after MSC transplantation in different animal models, which aim to avoid, hinder or reverse cartilage degradation. From a clinical point of view, a set of evidence has been demonstrated, concentrated mainly on the conditions predisposing to OA, such as chondral focal defects (CDF) and meniscal problems. In different protocols it has been observed that patients with CDF present an improvement in pain score, histological aspects and morphology observed by magnetic resonance imaging [11]. Patients with partial meniscectomy who were treated with MSC had 3.5 less risk of developing degenerative changes. In terms of local administration, several studies have shown that no serious adverse events have occurred after an average of 5 years of follow-up [12]

With regard specifically to knee osteoarthritis, in recent years, three pilot studies have shown safety and early evidence of clinical efficacy with a 6-month follow-up. These protocols are based on autologous therapy, with bone marrow MSC (BM) or adipose tissue (AD) [13].

However, so far there is little evidence to indicate the best source of cells for the treatment of knee osteoarthritis. Therefore, there is still a need for a composition for the effective treatment and / or prevention of this disease, such as the composition of the present invention.

Considering the aforementioned background, the inventors have developed a method of obtaining a specific population of mesenchymal cells from the umbilical cord, which have specific characteristics that allow them to be used for safe and effective cell therapies both in improving ejection volume of the heart in patients with heart failure, such as in reducing pain and improving functionality in joint wear injuries, also in the treatment of respiratory problems, such as lung lesions, and in the treatment of skin lesions, such as wounds chronic and ulcers.

### Description of the figures

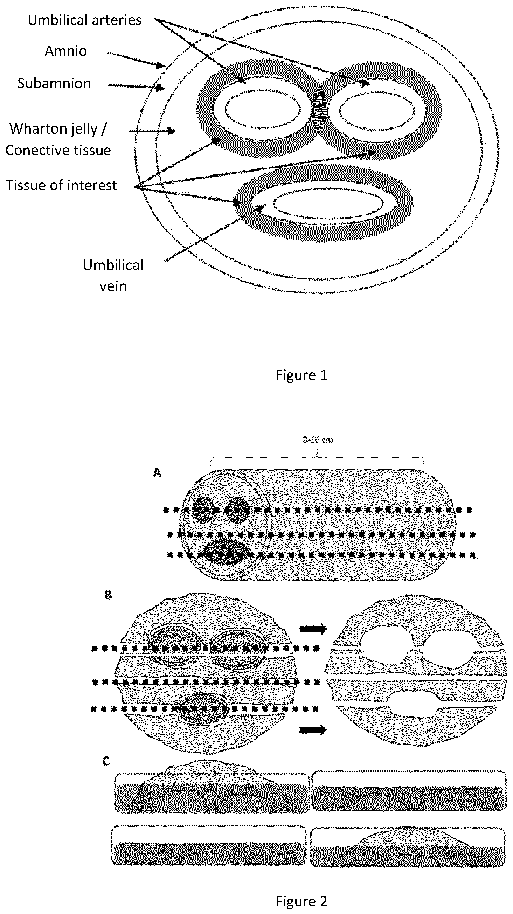

Figure 1: Transverse diagram of the human umbilical cord, which shows anatomical compartments.
Figure 2: Umbilical cord section. (A): Represents the three longitudinal cuts, once the amniotic and subamniotic layers have been removed. (B): Representation of connective tissue after removal of arteries and veins. (C): representation of the way of positioning the tissues on the plate with culture media.

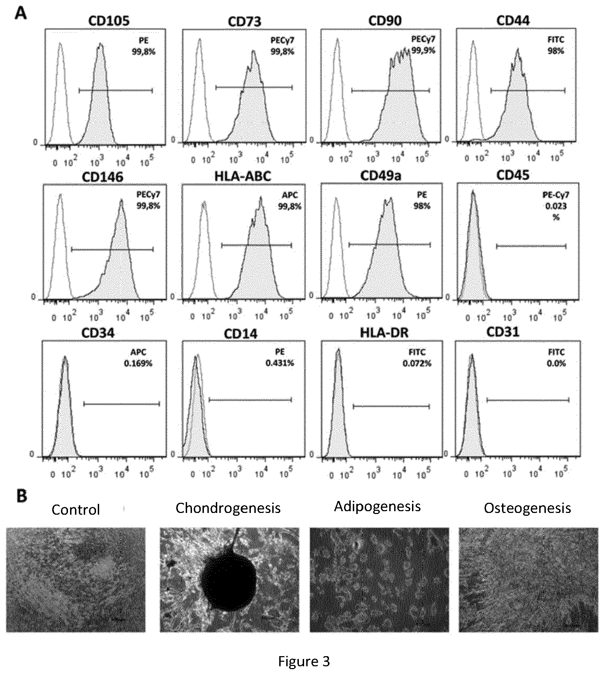

Figure 3: Cell characterization according to the guidelines of the International Society of Cell Therapy. The cellular composition derived from the umbilical cord expressed all common MSC markers and demonstrated the ability to differentiate into chondrogenic, osteogenic and adipogenic lineages. (A): The cell composition was analyzed by flow cytometry, samples from umbilical cord were stained with monoclonal antibodies labeled against known MSC surface markers (blue) and their respective isotypes (red). All umbilical cord derived cell compositions were positive for CD105, CD73, CD90, CD44, CD146 and CD49a, but negative for CD14, CD34, CD31, CD45 and HLADR. (B): Representative images of the differentiation of the cellular composition derived from the umbilical cord after specific inductions and stains: adipocytes (Oil Red O), osteocytes (alizarin red) and chondrocytes (Safranin O). Graduated bars = 200 mm. All data are presented as mean ± SEM (n = 3) of a minimum of 4 donors.

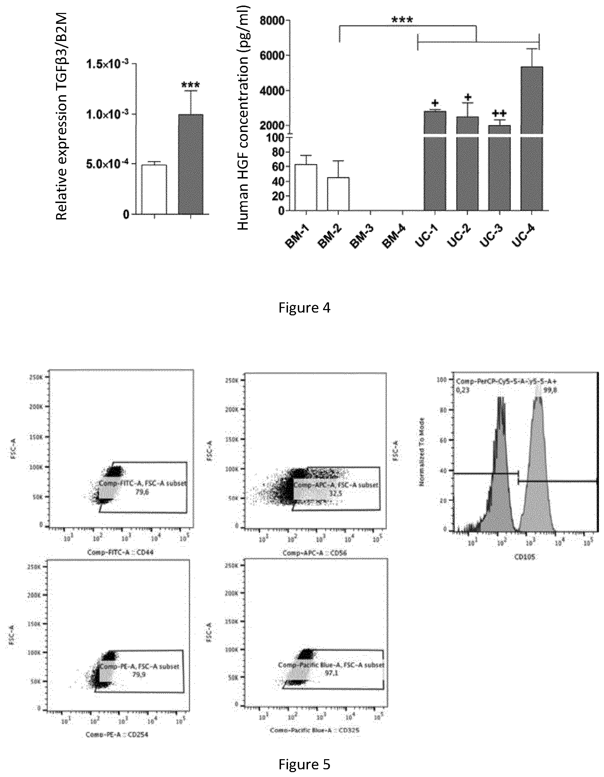

Figure 4: UC-MSCs and BM-MSCs showed a different profile in paracrine factors. TGFβ3 expression was quantified by RT-qPCR and HGF were measured by ELISA. HGF from different sources of the umbilical cord have a greater secretion than BM-MSC, which is of interest to be used as a method of selection. *** p < 0.001, + p <0.05, ++ p <0.001 UC-MSC-4 compared to UC-MSCs-1, 2 and 3.
Figure 5: Subpopulation characterization by special markers, approved cells (lot CU-108-6) have the markers CD44, CD56, CD105, CD254 (RANKL) and CD325 (N-cadherin).

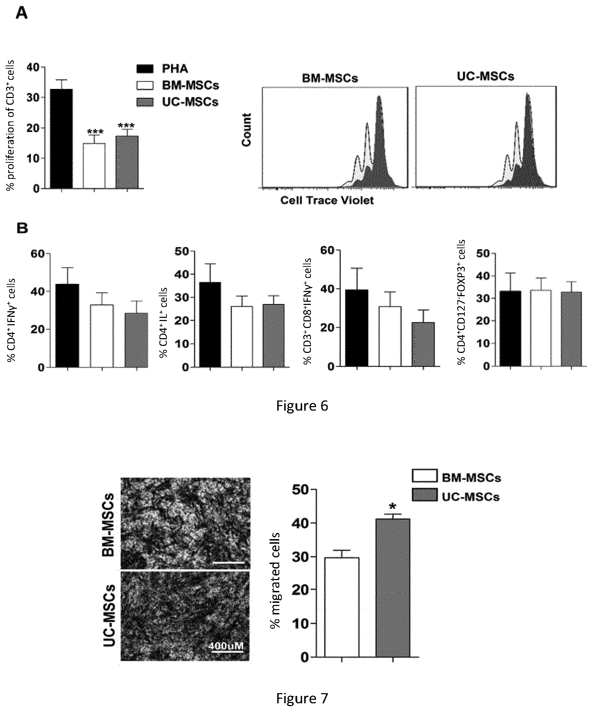

Figure 6: UC-MSCs and BM-MSCs show the same suppressor capabilities of proinflammatory T-cell inhibition. PHA-activated hPBMC obtained from patients with dilated cardiomyopathy with heart failure and reduced ejection fraction (HFrEF), marked with CFSE, were co-cultured with or without MSC in a 1:10 ratio (MSCs: hPBMC). (A): T cell proliferation was assessed by reducing the intensity of CFSE at 72 hours after culture, the graph on the left is a representation of the representative proliferation panel of CFSE (the light colored histogram represents activated PBMC and the histogram of dark color to activated PBMC co-cultured with MSCs). (B): Analysis of Th1, Th2, CD8 and regulatory T cells, from co-culture of PBMC and MSCs. The results are represented as mean ± SEM of at least 3 independent experiments using at least 3 different donors for hPBMC (healthy donor patient and DCM), UC-MSC and BM-MSC. *** p <0.001 UC-MSCs or BM-MSCs with respect to PHA.
Figure 7: The cellular composition derived from the umbilical cord has a superior migration capacity compared to BM-MSCs. The migration capacity of these cell types was assessed by the transwell assay in response to serum of patients with HFREF after 16 h. The images show the representative staining with violet crystal, while the graph shows the quantification of the% of migrated cells. The data shown in the graphs correspond to the mean ± SEM with at least three serum donors, UC-MSCs and BM-MSCs. * P <0.05 UC-MSC vs BM-MSCs.

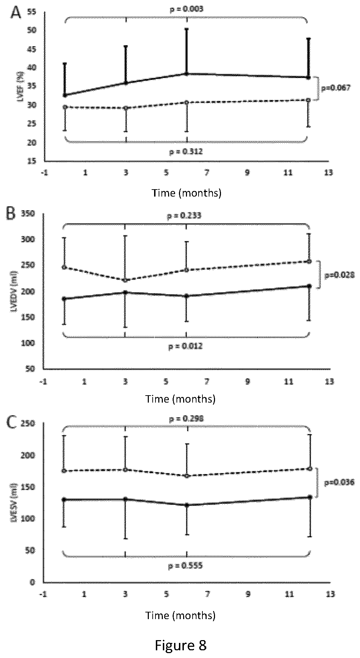

Figure 8: Changes in cardiac magnetic resonance measurements from baseline up to 12 months after treatment in the groups studied. (A): Ejection fraction of the left ventricle (LVEF). (B): Final diastolic volume of the left ventricle (LVEDV). (C): Final systolic volume of the left ventricle (LVESV). The solid line represents the group treated with the cellular composition derived from the umbilical cord (n = 14 per protocol). The dotted line represents the placebo group (n = 13 per protocol, withdrawal of consent from 1 patient). The statistical analysis was based on the regression of the maximum likelihood of mixed effect between the reference and follow-up measures for each group and the variability between the groups.

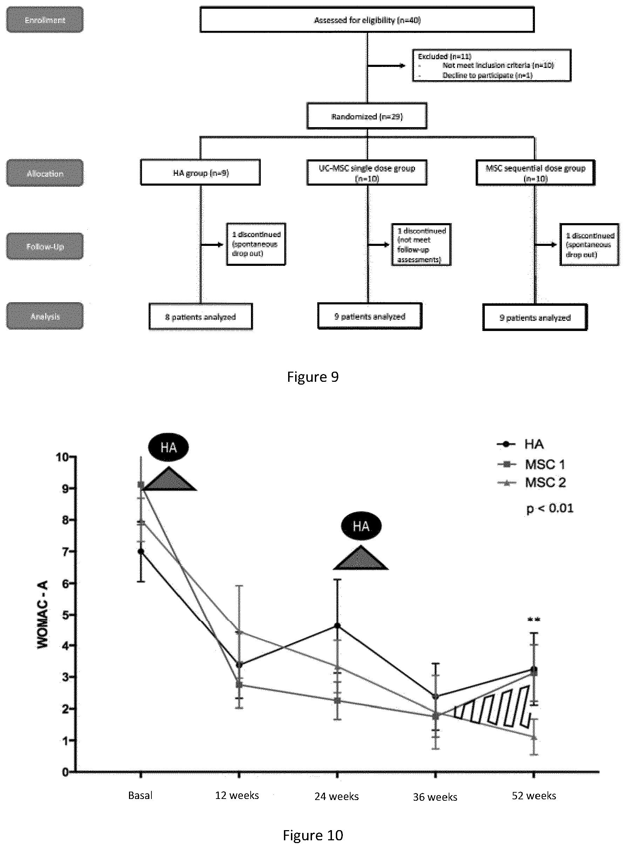

Figure 9: Design of the study and patient population for the Example "patients with interaction problems between the femur, patella and tibia".
Figure 10: WOMAC chart for patient pain from example 2 "Clinical study of patients with interaction problems between the femur, patella and tibia".

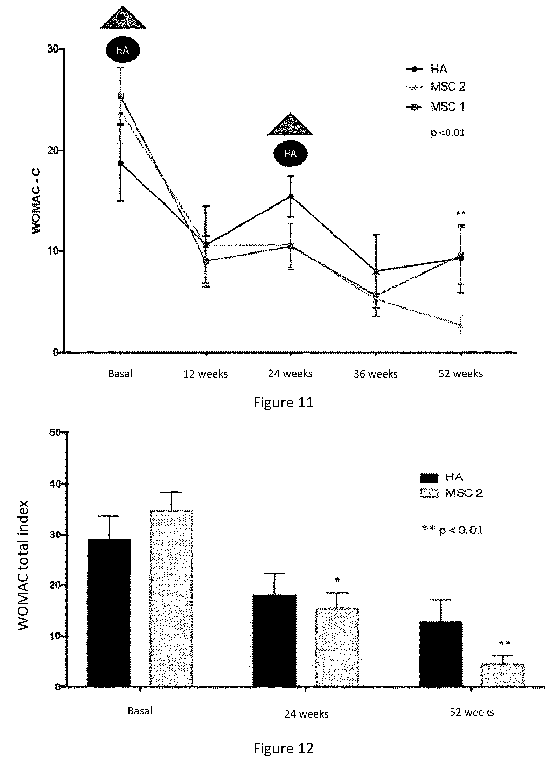

Figure 11: WOMAC chart for evaluation of patient functionality of example No. 2 "Clinical study of patients with interaction problems between the femur, patella and tibia".
Figure 12: Total Womac index graph for patients of example N° 2 "Clinical study of patients with interaction problems between the femur, the patella and the tibia".

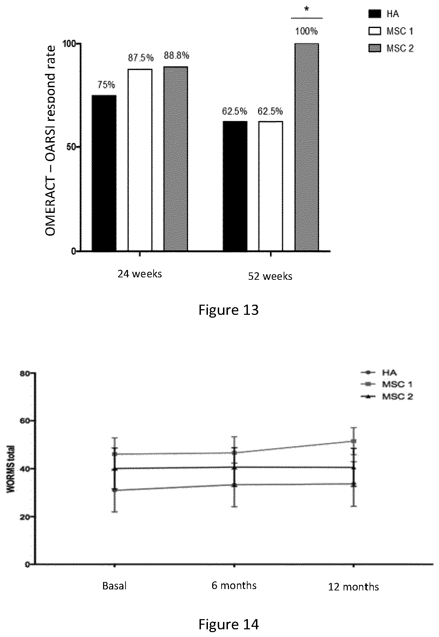

Figure 13: OMERACT - OARSI response index for patients of example N° 2 "Clinical study of patients with interaction problems between the femur, patella and tibia".
Figure 14: Structural result, through WORMS total for patients of example 2 "Clinical study of patients with interaction problems between the femur, the patella and the tibia".

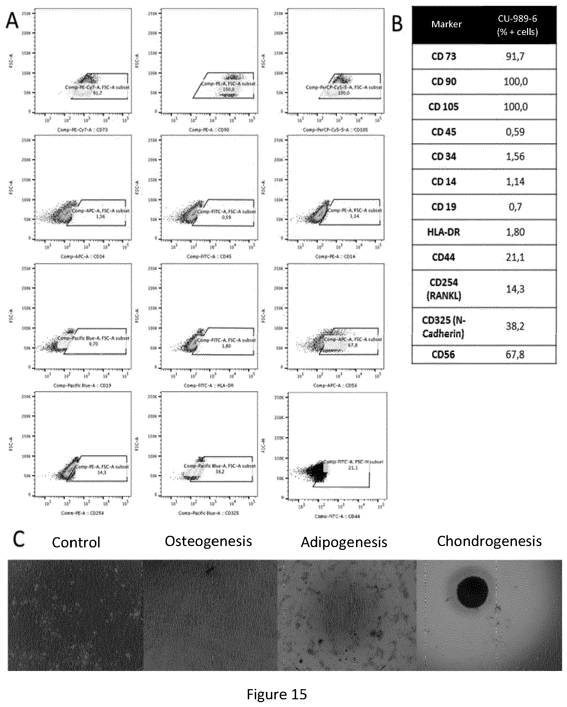

Figure 15: Cell characterization according to the guidelines of the International Society of Cell Therapy. The CU-989-6 cell composition derived from the umbilical cord expressed all common MSC markers and demonstrated the ability to differentiate into chondrogenic, osteogenic and adipogenic lineages. (A): The cell composition CU-989-6 was analyzed by flow cytometry, the samples from the umbilical cord were stained with monoclonal antibodies against known MSC surface markers and their respective isotypes. All umbilical cord derived cell compositions were positive for CD73, CD90, CD105 and negative for CD45, CD34, CD14, CD19 and HLA-DR. (B): Table with percentage of presence of the markers described above plus the markers of the selection criteria presented in this patent for markers CD44, CD56, CD105, RANKL and N-cadherin. (C): Representative images of the differentiation of the cellular composition derived from the umbilical cord after specific inductions and stains: osteocytes (alizarin red), adipocytes (Oil Red O) and chondrocytes (Safranin O).

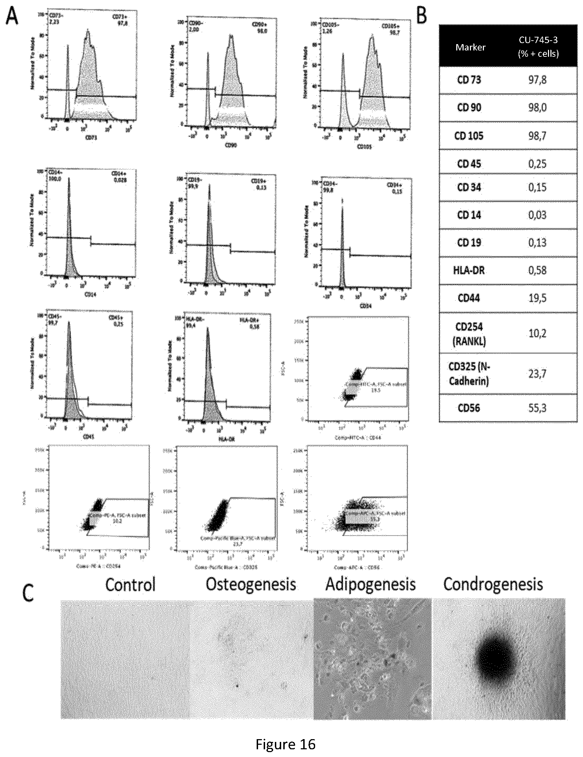

Figure 16: Cell characterization according to the guidelines of the International Society of Cell Therapy. The CU-745-3 cell composition derived from the umbilical cord expressed all common MSC markers and demonstrated the ability to differentiate into chondrogenic, osteogenic and adipogenic lineages. (A): The CU-745-3 cell composition was analyzed by flow cytometry, samples from umbilical cord were stained with monoclonal antibodies against known MSC surface markers and their respective isotypes. All umbilical cord derived cell compositions were positive for CD73, CD90, CD105 and negative for CD45, CD34, CD14, CD19 and HLA-DR. (B): Table with percentage of presence of the markers described above plus the markers of the selection criteria presented in this patent for markers CD44, CD56, CD105, RANKL and N-cadherin. (C): Representative images of the differentiation of the cellular composition derived from the umbilical cord after specific inductions and stains: osteocytes (alizarin red), adipocytes (Oil Red O) and chondrocytes (Safranin O).

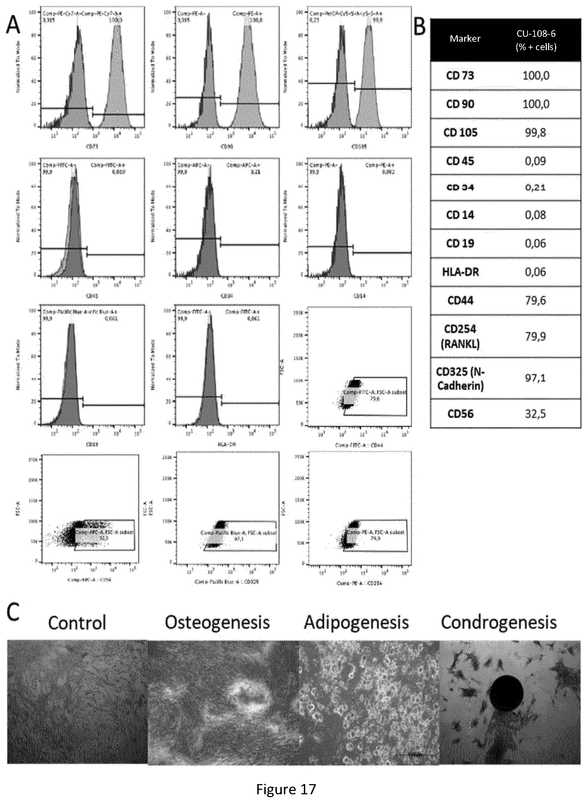

Figure 17: Cell characterization according to the guidelines of the International Society of Cell Therapy. The CU-108-6 cell composition derived from the umbilical cord expressed all common MSC markers and demonstrated the ability to differentiate into chondrogenic, osteogenic and adipogenic lineages. (A): The CU-108-6 cell composition was analyzed by flow cytometry, samples from umbilical cord were stained with monoclonal antibodies against known MSC surface markers and their respective isotypes. All umbilical cord derived cell compositions were positive for CD73, CD90, CD105 and negative for CD45, CD34, CD14, CD19 and HLA-DR. (B): Table with percentage of presence of the markers described above plus the markers of the selection criteria presented in this patent for markers CD44, CD56, CD105, RANKL and N-cadherin. (C): Representative images of the differentiation of the cellular composition derived from the umbilical cord after specific inductions and stains: osteocytes (alizarin red), adipocytes (Oil Red O) and chondrocytes (Safranin O).

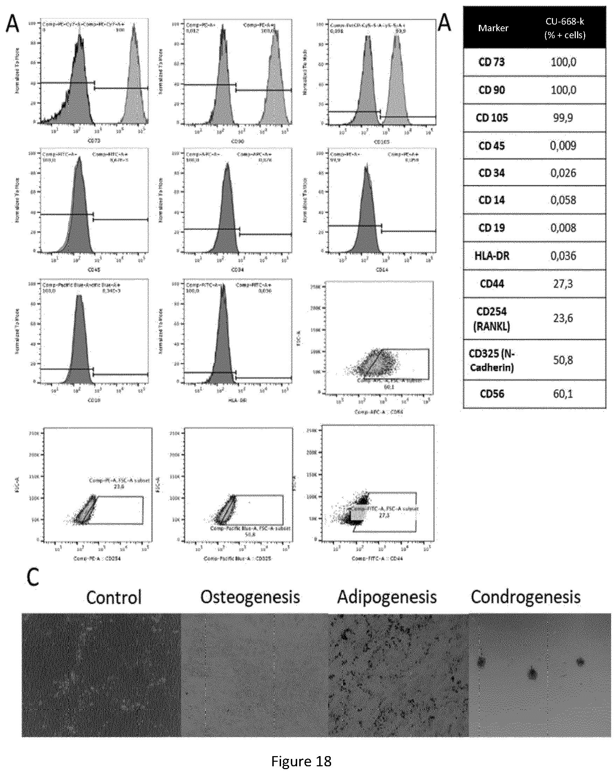

Figure 18: Cell characterization according to the guidelines of the International Society of Cell Therapy. The CU-668-K cell composition derived from the umbilical cord expressed all common MSC markers and demonstrated the ability to differentiate into chondrogenic, osteogenic and adipogenic lineages. (A): The cell composition CU-668-K was analyzed by flow cytometry, the samples from the umbilical cord were stained with monoclonal antibodies against known MSC surface markers and their respective isotypes. All umbilical cord derived cell compositions were positive for CD73, CD90, CD105 and negative for CD45, CD34, CD14, CD19 and HLA-DR. (B): Table with percentage of presence of the markers described above plus the markers of the selection criteria presented in this patent for markers CD44, CD56, CD105, RANKL and N-cadherin. (C): Representative images of the differentiation of the cellular composition derived from the umbilical cord after specific inductions and stains: osteocytes (alizarin red), adipocytes (Oil Red O) and chondrocytes (Safranin O).

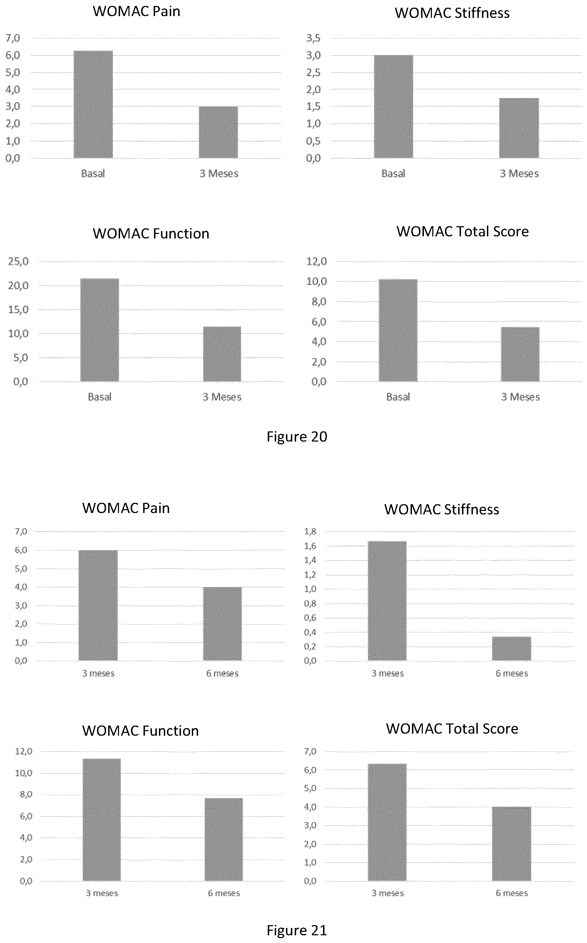

Figure 20: Results of the treatment of 4 patients with the cell composition CU-745-3.
Results presented according to the Index the Western Ontario and McMaster Universities Osteoarthritis Index (WOMAC) for a comparison of baseline status before treatment versus evaluation 3 months after the treatment was administered.
Figure 21: Results of the treatment of 3 patients with the cell composition CU-745-3.
Results presented according to the Index the Western Ontario and McMaster Universities Osteoarthritis Index (WOMAC) for a comparison of the patient's condition at 3 months of treatment versus evaluation at 6 months of administered the treatment.

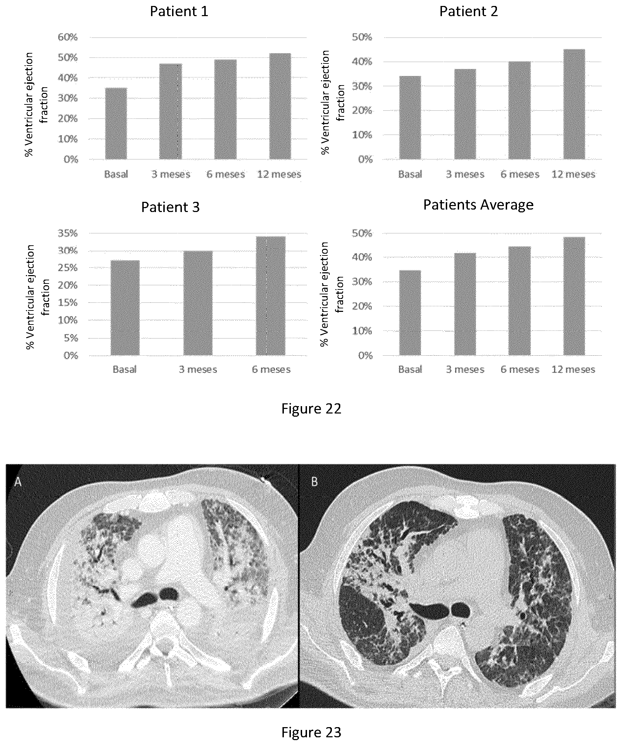

Figure 22: Results of the treatment of 3 patients with the cellular compositions CU-108-6 and CU-745-3 during the period of 1 year. Results presented according to the percentage of ventricular ejection fraction.
Figure 23: Effectiveness of cell therapy for the treatment of acute respiratory distress syndrome. (A): Computerized thoracic tomography (carinal section) of a patient suffering from acute respiratory distress syndrome before being treated with the cellular composition obtained using the methodology described in this document. (B) Thoracic computed tomography (carinal section) of the patient 13 days after being treated with the cell composition selected with the methodology of the invention.

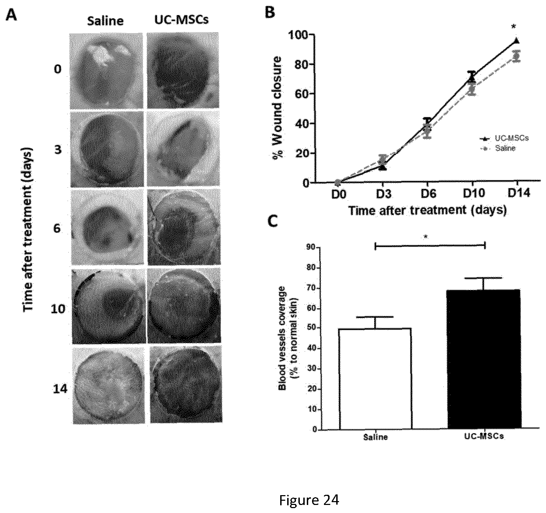

Figure 24: Evaluation of the improvement of skin regeneration through the use of cell compositions 108-6 and 745-3, selected by the method described herein. (A): Representative images of the wound evaluated at different times. (B): Percentage of wound closure at different times, the percentage of closure was calculated using the software Image J. (C): Quantification of angiongenesis in biopsies after 14 days, the analysis was developed using the software VesSeg-Tool. The values represent the mean ± standard error and to compare the significance of the group of cellular compositions vs. the saline group was used t-test (Mann Whitney), * p ≤ 0.05. UC-MSC: cellular compositions obtained by the method of the invention.

### Summary of the invention

The present invention is related to a method of obtaining a subpopulation of umbilical cord mesenchymal stem cells, optionally selected from the connective tissue that exists around the umbilical arteries, veins and the inner face of the subamniotic tissue. A composition containing this population of specific cells is useful for preparing a medicament that is used for cell therapies, such as problems with cardiac ejection flow of patients with a decreased ejection fraction. At the same time, this subpopulation is useful for preparing a medication that serves to relieve pain and recover the functionality of joint wear. And it is also useful for treating respiratory problems, such as lung lesions, and for treating skin lesions, such as chronic wounds and ulcers.

Method of obtaining a composition containing a specific population of umbilical cord mesenchymal cells comprising the following stages:
a) Cultivate and expand umbilical cord mesenchymal cells,
b) Select them by the presence of at least 3 of the N-cadherin, CD44, RANKL, CD105 and CD56 markers.

Where, optionally after stage b) a previous selection is made by production of hepatocyte growth factor (HGF), selecting cultures that secrete more than 1000 pg / mL of HGF in the culture medium; preferably more than 1500 pg / mL, or more than 2000 pg / mL and most especially more than 2500 pg / mL of HGF in the culture medium.

In addition, optionally after step b), an optional selection is made by TGF-β3 expression, the cultures that express the highest levels of relative expression being selected in comparison to the samples evaluated.

In a preferred embodiment, optionally the umbilical cord mesenchymal cells are specifically obtained from the connective tissue cells that surround the arteries and the umbilical vein and that is adjacent to the inner face of the subaminotic layer. In this embodiment, the amniotic and subamniotic layers are first removed in the umbilical cord, second, 3 longitudinal and parallel cuts are made through the arteries and the umbilical vein so as to expose the vein and arteries, and remove them from the tissue. Where the cuts obtained clean of arteries and veins are plated with culture medium so that the connective tissue that was adjacent to the vein and arteries is in direct contact with the culture plate.

On the other hand, optionally in step a) the cells are cultured for at least 8 days, or at least 10 days.

To obtain the mesenchymal cells, the cultured cells are selected by cytometry, according to their markers by selecting as mesenchymal cells those that express more than 95% CD105, CD73 and CD90 and that at the same time do not express, that is, express less than 2% CD45, CD34, CD14 and HLA-DR. Subsequently, the selected mesenchymal cells expand and separate according to what is established in stage b).

Finally, in step b) the cells are selected because they express N-cadherin and at least 2 of the markers, CD105, CD44, RANKL or CD56. More especially, the cells are selected because they express N-cadherin, RANKL and at least 1 of the markers, CD105, CD44, or CD56. Preferably, the cells are selected because they express N-cadherin, RANKL and at least 2 of the markers, CD105, CD44, or CD56, and most preferably the cells are selected because they express N-cadherin, RANKL, CD105, CD44, and CD56.

The composition containing a specific population of umbilical cord mesenchymal cells comprising mesenchymal cells expressing N-cadherin and at least 2 of the markers, CD105, CD44, RANKL or CD56, and which is obtained by the method of invention.

The invention also aims at the use of the composition containing cells from a specific population of umbilical cord mesenchymal cells obtained according to the method described to prepare a medicament useful for cell therapies. Especially to prepare a useful medication to treat problems of cardiac ejection flow. More especially, to prepare a medicament useful for treating problems of cardiac ejection flow that is administered intravenously. Where it preferably serves to prepare a drug useful for treating heart failure.

In another embodiment, the composition obtained by the method of the invention is useful for preparing a medicament useful for treating pain and improving functionality associated with problems of joint wear. Especially to treat wear in the interaction between the femur, tibia and patella; or to treat wear between the head of the femur with the acetabulum; or to treat wear between the talus, tibia and fibula; or to treat wear between the radius, cube and carpal / carpal bones, or to treat wear on knee joints. That is, the composition of the invention serves to prepare a medicament useful for treating wear on joints of the ankle, hip, knee, wrist, shoulder, fingers, elbow neck and spine.

On the other hand, the composition obtained by the method of the invention is useful for preparing a medicament useful for treating respiratory problems, such as lung lesions, acute lung lesions, such as acute respiratory distress syndrome and for treating skin lesions, such as chronic wounds and ulcers.

### Detailed description of the preferred embodiment

### Definitions

In the context of the present invention, the term "mesenchymal stem cells" ("MSCs") refers to multipotent progenitor cells derived from tissue-mesoderm. Which have the ability to differentiate into cells that make up adipose, bone, cartilaginous and muscular tissues. The minimum criteria established by the International Society of Cell Therapy present that to ensure the identity of MSC cells must be positive for the CD73, CD90 and CD105 markers, and negative for the CD45, CD34 and CD14 markers. MSCs can be found in almost all tissues of the human body.

As used in the present application, "pharmaceutically acceptable carrier" or "pharmaceutically acceptable diluent" means any and all solvents, dispersion media, coatings, antibacterial and antifungal agents, isotonic agents and absorption retardants, compatible with pharmaceutical administration. The use of such medias and agents for pharmaceutically active substances is well known in the art. Acceptable vehicles, excipients or stabilizers are not toxic to the receptors at the dosages and concentrations employed and, without limiting the scope of the present invention, include: additional buffering agents; Preservatives; Co-solvents; Antioxidants, including ascorbic acid and methionine; Chelating agents such as EDTA; Metallic complexes (for example, Zn protein complexes); Biodegradable polymers, such as polyesters; Salt forming counterions, such as sodium, polyhydric sugar alcohols; Amino acids, such as alanine, glycine, glutamine, asparagine, histidine, arginine, lysine, ornithine, leucine, 2-phenylalanine, glutamic acid and threonine; Organic sugars or sugar alcohols, such as lactitol, stachyose, mannose, sorbose, xylose, ribose, ribitol, myoinisitose, myoinisitol, galactose, galactitol, glycerol, cyclosols (eg, inositol), polyethylene glycol; Sulfur-containing reducing agents, such as urea, glutathione, thioctic acid, sodium thioglycolate, thioglycerol, α-monothioglycerol and sodium thio sulfate; Low molecular weight proteins, such as human serum albumin, bovine serum albumin, gelatin or other immunoglobulins; And hydrophilic polymers, such as polyvinylpyrrolidone.

The terms "treatment" and "therapy", as used in the present application, refer to a set of hygienic, pharmacological, surgical and / or physical means used with the intention of curing and / or alleviating a disease and / or symptoms with the aim of remedying the health problem. The terms "treatment" and "therapy" include preventive, curative and paleative methods, since both are aimed at maintaining and / or restoring the health of an individual or animal. Regardless of the origin of the symptoms, disease and disability, the administration of a suitable medication to alleviate and / or cure a health problem should be interpreted as a form of treatment or therapy in the context of this application.

The term "prevention", as used in the present application, refers to a set of hygienic, pharmacological, surgical and / or physical means used to prevent the onset and / or the development of a disease and / or symptoms. The term "prevention" encompasses prophylactic methods, since these are used to maintain the health of an animal or individual.

The terms "individual", "patient" or "subject" are used interchangeably in this application and are not intended to be limiting in any way. The "individual", "patient" or "subject" can be of any age, sex and physical condition.

The term "therapeutically effective amount" refers to an amount of active component and / or cells that have a therapeutic effect and is capable of preventing and / or treating a disease and / or injury.

The term "heart failure" refers to the inability of the heart to keep up with its requirements and, specifically, the failure of the heart to pump blood with normal efficiency. In a preferred embodiment, the heart failure is chronic heart failure with reduced ejection fraction.

The term "heart failure with reduced ejection fraction" refers to a heart condition in which the patient suffers from a deficiency in the amount of blood expelled by the heart, usually documented by a left ventricular ejection fraction of 40% or less on echocardiography.

The term "chronic" refers to a condition and / or disease that persists for a long time or that is constantly repeated.

The inventors have developed a method to obtain the population of cells from the umbilical cord, which are especially suitable for the treatment of ventricular ejection problems and problems of wear on the joints, especially in wear due to the interaction between the femur, the patella and tibia. And also useful for treating respiratory problems, such as lung lesions, and for treating skin lesions, such as chronic wounds and ulcers".

In general terms, the method of the invention comprises specifically obtaining the connective tissue cells that surround the arteries and the umbilical vein and which is adjacent to the inner face of the subaminotic layer of the umbilical cord; cultivate and expand the cells obtained, validate that these are mesenchymal cells and also the cells respond to at least 3 of the N-cadherin, CD44, RANKL, CD105 and CD56 markers. Optionally, but preferably after selection by the indicated markers, a selection is made by production of hepatocyte growth factor (HGF), selecting cell cultures that secrete more than 1000 pg / mL of HGF. In parallel to this last stage, an evaluation of the expression of TGF-β3 is performed by RT-qPCR.

As indicated in the background of the invention, different protein expression patterns, such as growth factors and surface markers (CDs) are related to the differentiation and potential of these cells when used, for example in cell therapies. The inventors have determined that mesenchymal cells, obtained from certain areas of connective tissue within the umbilical cord, as detailed below and that at the same time express at least 3 of 5 markers selected from N-cadherin, CD44, RANKL, CD105 and CD56 , and that optionally secrete high amounts of hepatocyte growth factor (HGF) and TGF-β3 expression have a better result when used in different cell therapies. Specifically, these cells are useful in therapies to provide a better result in improving ventricular ejection problems, joint wear, to treat respiratory problems, and to treat skin lesions.

These molecules selected as markers of the subpopulation of cells of the invention can induce or block differentiation lines:
- N-cadherin is a transmembrane protein that has a role in cell-cell communication. This protein has also been linked to cell adhesion and is involved in cardiomyogenic differentiation [14]. It has also been described as an important modulator, due to its change in the expression profile, for the osteogenesis of mesenchymal stem cells from bone marrow [15,16].

- CD44 is a marker of proteins involved in cell growth, survival and differentiation of h-MSCs [17]. This molecule has been presented as a candidate for the activation of the chondrogenic pathway in 3-D matrices in interaction with hyaluronic acid [18].
- RANKL has been related as an important protein in osteoclastogenesis, therefore, when present it is able to inhibit the osteogenic pathway [19].
- CD105, despite being a canonical marker in h-MSC, is an important co-receptor for signaling the transforming factor of beta-type tissue (TGF-β) and has also been related to paracrine stimulation of angiogenesis by cardiac stromal cells [20].
- CD56 is a surface protein that has reported that in combination with other markers such as MSCA-1, they have a greater potential for differentiation to the chondrogenic and pancreatic pathway than when it is not present. This protein has also been implicated in cell migration, cell-cell and cell-matrix interaction. The expression profile that this molecule has in the heart suggests that it may be modulating different events such as the interaction between different cell types.

Additionally, these 5 markers are also useful for selecting cells that help improve problems with respiratory injuries. These molecules selected as markers of cell subpopulation can modulate responses as follows:
- CD44: is a marker present in extracellular vesicles used by mesenchymal cells to promote an anti-inflammatory response and high phagocytic activity to treat respiratory injury models such as Acute Respiratory Distress Syndrome (ARDS) [27]
- RANKL: It has been described that the expression of RANKL in mesenchymal cells regulates class II molecules of the major complex of hypertropability (MHC II), which interferes with the inflammatory response [28].
- CD105: Studies have validated the use of CD105 positive cells for the treatment of respiratory lesions such as ALI or its more severe ARDS form [29]. Additionally, it has been presented the role that plays this marker in angiogenesis, endothelial cell proliferation and vasculature development [30]; This is essential for the repair of the gas exchange interface in a patient who has suffered from ARDS [31].
- CD56: Considering the distribution of infused MSC cells, it houses these cells primarily in the lung [32], and that CD56 is a marker expressed by mesenchymal stem cells that is related to cell-cell and cell-matrix interactions [33], presence of this marker would promote interaction with affected tissues in the lung.
- N-Cadherin: For mesenchymal cells it has been described that N-cadherin is responsible for the regulation of Vascular Endothelial Growth Factor (VEGF), crucial factor in the formation of vessels (angiogenesis) [34]. Additionally, the therapeutic importance of MSCs by means of VEGF for treating lung lesions has been described [35]. Therefore, the presence of the N-Cadherin marker in MSC plays a role in the treatment of lung lesions

Additionally, these 5 markers are also useful for selecting cells that help improve problems with skin wounds. These molecules selected as markers of cell subpopulation can modulate responses as follows:
- CD44: It is a cell surface adhesion receptor that has been used to identify stem cells [36], specifically the expression of this marker plays a fundamental role in tissue regeneration for skin wounds due to cell migration [37, 38].
- RANKL: It has been described that the expression of RANKL in mesenchymal cells regulates class II molecules of the major complex of hypertropability (MHC II) [28]. This complex interacts directly with the mechanism of wound repair [39].
- CD105: This marker is related to angiogenesis, proliferation of endothelial cells and the development of vasculature [30], crucial elements for the treatment of wounds. Specifically, this is a marker is used by stem cells to regulate the inflammatory phase and repopulation of hematopoietic cells in the long term [40].
- CD56: is a marker expressed by mesenchymal stem cells that is related to cell adhesion through cell-cell and cell-matrix interactions [33], therefore the presence of this marker would promote MSC adhesion to the affected area.
- N-Cadherin: For mesenchymal cells it has been described that N-cadherin is responsible for the regulation of VEGF, crucial factor in the formation of vessels (angiogenesis) for tissue regeneration [3. 4]. Therefore, the presence of the N-Cadherin marker in MSC plays a role in wound treatment.

Human MSCs can secrete hepatocyte growth factor (HGF), this protein can act paracrine or autocrine, and can regulate cell growth, motility and morphogenesis in different types of cells and tissues [21]. HGF is synthesized and secreted in its precursor form, and is proteolytically activated in response to injury to some tissue such as liver, kidney, lung and stomach among others, thus participating in tissue repair and regeneration, having relevance in blood clotting and in the fibrinolytic system [21]. For example, it has been described that HGF is present in mediating the response in wound healing and cardioprotection [23].

Additionally, human MSCs can also produce the transforming growth factor beta-3 (TGF-β3), this factor is a protein of the cytokine family that is involved in embryogenesis, development and cell differentiation. Studies have shown that the presence of TGF-β3 helps improve the synthesis of type II collagen and glycosoaminoglycan (GAGs) of chondrocytes, and that this synthesis helps differentiate UC-MSC to chondrocytes [24]. On the other hand, TGF-β3 is also known for its antifibrotic activity [25] and its role in tissue regeneration [26].

Although all the selected markers are known, there is currently no document that anticipates a method of obtaining a subpopulation of cells with these characteristics, nor is it obvious that the presence of at least 3 of the N-cadherin, CD44, RANKL, CD105 and CD56 markers correlates with greater effectiveness in therapies with these cells, not least for treatment and / or prevention of ejection deficiencies in patients with heart failure, and can also be used to relieve pain and improve functionality in patients with joint wear problems; to treat respiratory problems, such as lung injuries, acute lung injuries, such as acute respiratory distress syndrome and to treat skin lesions, such as chronic wounds and ulcers.

### Cord Processing

To perform the method of the invention the umbilical cords are obtained from full-stage human placentas extracted by caesarean section after informed consent, obtained from healthy donors and stored aseptically in an antibiotic buffer solution.

The umbilical cord is stored in a sterile buffer such as Hank's biocarbonate buffer or phosphate buffered saline (PBS). More preferably, the umbilical cord is stored in a PBS solution.

As indicated the umbilical cord is treated with antibiotics, such as penicillin and streptomycin at a concentration between 50-300 units / ml and 50-300 µg / ml, respectively. More preferably, the umbilical cord is treated with penicillin and streptomycin at 100 units / ml and 100 µg / ml, respectively.

In a preferred embodiment, the umbilical cord should be sectioned according to the method of the invention and washed with the buffer solution and antibiotics in a time not exceeding 5 hours. More preferably,the procedure should not exceed 4 hours. More preferably, the sectioning and washing procedure should not take more than 3 hours.

In an optional embodiment, the tissue of interest of the invention is located within the layer that exists around the umbilical arteries, the umbilical vein and the outer face of the connective tissue was in contact with the subamniotic layer, as shown in the Figure 1. Once the umbilical cord is cross-sectioned into small sections of 8 to 10 cm, the amniotic and subamniotic layers are removed to expose the connective tissue that contains the vein and umbilical arteries.

As shown in Figure 2A, through three parallel longitudinal cuts of the connective tissue, the vein and umbilical arteries are exposed and removed as shown in Figure 2B, then the layers surrounding these arteries and the umbilical vein are seeded in culture plates and maintained in stabilization culture media, as shown in Figure 2C. It is important that the tissue of interest is in direct contact with the culture plate.

The stabilization culture medium may be composed of any of the following elements such as culture of cell media, sugars, antibiotics, antifungals and amino acids. Preferably, the culture medium should contain culture mediums such as DMEM-F12, DMEM with or without high glucose concentration, Alpha-MEM with or without high glucose concentration, antibiotics such as penicillin and streptomycin, antifungals such as amphotericin, and amino acids such as L-glutamine. More preferably, the culture should contain 10 ml of Eagle's minimum essential medium (MEM), alpha modifications (Alpha-MEM) with high glucose connection (Gibco, Gran Island, USA) supplemented with 10% heat-inactivated fetal bovine serum (FBS; Gibco, USA), 1% penicillin / streptomycin and 2 mM L-glutamine (LG) (Gibco, Gran Island, USA).

### Isolation of cell subpopulation by explant

To obtain a population of cells that exist within the layer that surrounds the umbilical arteries and veins, the tissue must be seeded in culture plates as shown in Figure 2C, bringing the layer surrounding the arteries and umbilical veins into the bottom. internal plate. This explant technique allows obtaining a population of cells capable of migrating and adhering to the plaque from the connective tissue. With standard times for cell adhesion of 10 to 15 days, the population of cells capable of migrating and adhering to the plate would come from all connective tissue. With a different approach, the objective of the technique presented in this invention is to obtain only the cellular population that exists within the layer that surrounds the umbilical arteries and veins, therefore, the migration / adhesion time must be shorter than the techniques usual explant. In a preferred embodiment, the time to allow the cells to migrate out of the connective tissue and adhere to the inner bottom of the plaque should not be more than 10 days. More preferably, the time for cell migration and adhesion should not be longer than 8 days.

Within the first 48 hours it is likely that the tissue surrounding the arteries and umbilical veins will adhere to the plaque, once this happens, the stabilization culture medium should be replaced with fresh medium every 3 days, dividing the culture continuously until passage 3.

This procedure to obtain a population of cells that exist within the layers surrounding the umbilical arteries and veins, must be repeated in the same way to obtain the cells that are within the tissue of the outer face of the connective tissue, which was surrounded by the subamniotic tissue

Once the cell culture has reached a confluence of 70-80%, the cells must be separated from the plaque and reseeded in separate bottles in accordance with the tissue segment from which it was obtained. In a preferred embodiment, the cells can be separated from the plate by TrypLE ™ Express (Gibco, Gran Island, USA), and subsequently seeded at a density of 2,500 cells per cm² in 500 cm² flasks (Nunc, Denmark) identifying the segment of the tissue from which it was extracted.

Cell cultures should be taken to passage 3 to be later characterized according to the guidelines of the International Society of Cell Therapy [1].

Characterization according to the guidelines of the International Society of Cell Therapy In a preferred embodiment, the characterization should be carried out according to the guidelines of the International Society of Cell Therapy using cytometry for the markers CD105, CD73, CD90, CD45, CD34, CD14, HLA-DR and in vitro differentiation in osteoblasts, adipocytes and chondroblasts, as shown in Figure 3.

In a preferred embodiment, at least 90% of the cells in the composition are CD105 positive. Preferably, at least 91, 92, 93 or 94% of the cells are CD105 positive. More preferably, 95% or more of the cells are CD105 positive.

In a preferred embodiment, at least 90% of the cells of the composition are CD73 positive. Preferably, at least 91, 92, 93 or 94% of the cells are CD73 positive. More preferably, 95% or more of the cells are CD73 positive.

In a preferred embodiment, at least 90% of the cells of the composition are CD90 positive. Preferably, at least 91, 92, 93 or 94% of the cells are CD90 positive. More preferably, 95% or more of the cells are CD90 positive.

In a preferred embodiment, at least 90% of the cells in the composition are CD105, CD73 and CD90 positive. Preferably, at least 91, 92, 93 or 94% of the cells are CD105, CD73 and CD90 positive. More preferably, 95% or more of the cells are CD105, CD73 and CD90 positive.

In a preferred embodiment, less than 10% of the cells in the composition are CD45 positive. Preferably, less than 10, 9, 8, 7, 6, 5, 4 or 3% of the cells are CD45 positive. More preferably, 2% or less of the cells are CD45 positive.

In a preferred embodiment, less than 10% of the cells of the composition are CD34 positive. Preferably, less than 10, 9, 8, 7, 6, 5, 4 or 3% of the cells are CD34 positive. More preferably, 2% or less of the cells are CD34 positive.

In a preferred embodiment, less than 10% of the cells in the composition are CD14 positive. Preferably, less than 10, 9, 8, 7, 6, 5, 4 or 3% of the cells are CD14 positive. More preferably, 2% or less of the cells are CD14 positive.

In a preferred embodiment, less than 10% of the cells of the composition are HLA-DR positive. Preferably, less than 10, 9, 8, 7, 6, 5, 4 or 3% of the cells are positive for HLA-DR. More preferably, 2% or less of the cells are positive for HLA-DR.

In a preferred embodiment, less than 10% of the cells of the composition are CD45, CD34, HLA-DR and / or CD14 positive. Preferably, less than 10, 9, 8, 7, 6, 5, 4 or 3% of the cells are CD45, CD34, HLA-DR and / or CD14 positive. More preferably, 2% or less of the cells are CD45, CD34, HLA-DR and / or CD14 positive.

In a preferred embodiment, at least 70% of the cells of the composition are viable. Preferably, at least 71, 72, 73, 74, 75, 76, 77, 78 or 79% of the cells are viable. More preferably, 80% or more of the cells are viable.

In a preferred embodiment, the composition does not comprise macroscopic aggregates, contamination by pathogenic microorganisms (bacteria, mycoplasma, syphilis, HBV, HCV, HIV, CMV and fungi) and an endotoxin concentration above 0.5 EU / ml.

The cells obtained by this procedure can be considered as mesenchymal cells suitable for cell therapy.

### MSC subpopulation characterization

Once the cells have been validated as mesenchymal cells, the inventors have developed a selection of cell cultures based on 5 markers by cytometry. These cell cultures will be selected by the presence of at least 3 of the markers CD105, CD56, CD44, RANKL and N-cadherin. The inventors have established that the cells that respond to these markers are especially suitable for the treatment of ventricular ejection problems and joint wear problems, for example in the interaction between the femur, the patella and the tibia; to treat respiratory problems, such as lung injuries, acute lung injuries, such as acute respiratory distress syndrome and to treat skin lesions, such as chronic wounds and ulcers.

The inventors have characterized these cells in detail, cells that have any of the following characteristics are more likely to be effective in the treatment of ventricular ejection deficiency of the heart and in relieving pain and recovering the functionality of the wear generated in the interaction between the femur, the patella and tibia; to treat respiratory problems, such as lung injuries, acute lung injuries, such as acute respiratory distress syndrome and to treat skin lesions, such as chronic wounds and ulcers.

To begin with the subpopulation characterization procedure, cell cultures obtained from MSC validation have to be washed, resuspended and incubated. Most preferably, cell cultures should be washed 4 times successively with sterile PBS, at pH 7.3 and 4 ° C.

Once the washing is finished, the cell cultures should be resuspended, in a preferred embodiment they are resuspended at a concentration of 1 * 10⁶ solution with sterile PBS at pH 7.3.

After mixing, the cell cultures should be incubated in a preferred embodiment, incubated for 30 min on ice and dark with a mixture of the primary antibodies against N-caderin, CD44, RANKL, CD105 and / or CD56.

To place the sample in the cytometer, cell cultures should be resuspended with 10 ml of sterile PBS at pH 7.3 in sterile 12x75 mm propylene tubes.

For cytometry, more preferably a BD FACS Canto II flow cytometer should be used. Most preferably, the subpopulation should express CD44, CD56, CD105, N-cadherin and RANKL or a combination of these markers.

In a preferred embodiment, for cell cultures to be approved, at least 10% of the cells in the composition are CD44 positive. Preferably, at least 10, 11, 12, 13 or 14% of the cells are CD44 positive. More preferably, 15% or more of the cells are CD44 positive.

In a preferred embodiment, for cell cultures to be approved, at least 15% of the cells in the composition are CD56 positive. Preferably, at least 15, 16, 17, 18 or 19% of the cells are CD56 positive. More preferably, 20% or more of the cells are CD56 positive.

In a preferred embodiment, for cell cultures to be approved, at least 50% of the cells in the composition are CD105 positive. Preferably, at least 91, 92, 93 or 94% of the cells are CD105 positive. More preferably, 95% or more of the cells are CD105 positive.

In a preferred embodiment, for cell cultures to be approved, at least 10% of the cells in the composition are N-cadherin positive. Preferably, at least 10, 11, 12, 13 or 14% of the cells are N-cadherin positive. More preferably, 15% or more of the cells are N-cadherin positive.

In a preferred embodiment, for cell cultures to be approved, at least 1% of the cells in the composition are RANKL positive. Preferably, at least 1, 2, 3 or 4% of the cells are RANKL positive. More preferably, 5% or more of the cells are RANKL positive.

In a preferred embodiment, for cell cultures to be approved, at least 10% of the cells in the composition are positive for at least 3 of the markers CD44, CD56, CD105, N-cadherin and RANKL. Preferably, at least 11, 12, 13, 14% of the cells are positive for 3 of the markers CD44, CD56, CD105, N-cadherin and RANKL. More preferably, 15% or more of the cells are positive for at least 3 of the markers CD44, CD56, CD105, N-cadherin and RANKL.

Cell cultures that approve the requirements of the International Society of Cell Therapy and the group of markers for the subpopulation mentioned above, can be subsequently evaluated by characterizing their extracellular products and their RNA expression.

### Subpopulation characterization according to levels of secreted factors

In a preferred embodiment, the supernatant from the different cell cultures should be collected and the secreted levels of Hepatocyte Growth Factor (HGF) should be measured, as shown in Figure 4. Selecting cell cultures that secrete more HGF.

In a preferred embodiment, the different supernatants should be measured using the ELISA DuoSet Development System (R&D Systems, Minneapolis, MN).

In a preferred embodiment, the cell culture that secretes more than 1000 pg / ml of hepatocyte growth factor (HGF) in the culture medium should be selected. Preferably, the culture of cells that secrete more than 1000, 1100, 1200, 1300, 1400, 1500, 1600, 1700, 1800, 1900, 2000, 2100, 2200, 2300, 2400, 2500, 2600, 2700, 2800 or 2900 pg / ml of HGF in the culture medium. More preferably, the cell culture that secretes more than 2500 pg / ml of HGF in the culture medium should be selected. Most preferably, the cell culture that secretes 3000 pg / ml or more in the culture medium should be selected.

### Subpopulation characterization according to gene expression levels

In a preferred embodiment, a sample of each cell culture should be taken and the expression of TGF-β3 evaluated by RT-qPCR, as shown in Figure 4. Selecting cultures that express a greater amount of TGF-β3.

In a preferred embodiment, TGF-β3 expression should be measured using the Universal SYBR Green Quantitative PCR Protocol (Merck) RT-qPCR kit.

Cell cultures that approve the aforementioned characterizations become mixed.

Once the final solution of the cell mixture is classified, the solution will be referred to as **"cellular composition derived from the umbilical cord",** this solution can be used immediately or it can be cryopreserved for later use. If a stored cellular composition has to be used, depending on the number of cells required in each treatment, the cryopreserved vials must be thawed and expanded. In a preferred embodiment, cryopreserved vials should thaw and expand to passage 5-6 using Alpha-MEM supplemented with 5% human platelet lysate (hPL).

### Examples

### Example 1. Obtaining subpopulation of cells of the invention.

To obtain the subpopulation of cells of the invention, an umbilical cord sample was taken from a full-stage human placenta, this placenta was extracted by caesarean section after the informed consent of a healthy donor; to subsequently be stored aseptically in a buffer solution with antibiotics, as described in detail in the "cord processing" section.

Subsequently, the cord was cross-sectioned into segments of 8-10 cm following the model presented in Figure 2A, then make three longitudinal cuts to expose and then remove the vein and umbilical arteries, as shown in Figure 2B. Position the inner faces surrounding the arteries and vein in contact with the bottom of the culture plates, as shown in Figure 2C. The cells that migrated and adhered to the plates were expanded to passage 3 with the medium detailed in the section "Isolation of the cellular subpopulation by explant".

The different plates obtained were validated by means of the protocol described in the section "Characterization of subpopulations of MSC". The results obtained for the markers CD105, CD73, CD90, CD45, CD34, CD14, HLA-DR and in vitro differentiation in osteoblasts, adipocytes and chondroblasts, are shown in Figure 3. Most of the plates approved this validation with higher percentages 98% for CD105, CD73, CD90 and less than 1% for CD45, CD34, CD14, HLA-DR.

Additionally, cell cultures that were approved according to the guidelines of the International Society of Cell Therapy were evaluated according to the group of markers CD105, CD56, CD44, RANKL and N-cadherin. The evaluated cell cultures marked presence for all the evaluated markers, with which they were approved to proceed to the next validation. Additionally, in this case the requirement that the evaluated cultures marked more than 15% for at least 3 of the markers, as shown in Figure 5, was also met.

For the approved cultures, the secretion of HGF in the supernatant of the different cell cultures was evaluated, as shown in Figure 4. The levels of HGF were greater than 2500 pg / ml for most of the cell cultures evaluated.

In parallel, the expression of TGF-β3 was evaluated by RT-qPCR, as shown in Figure 4. Cultures approved according to the evaluations described above showed high expression of TGF-β3.

Finally, the cell cultures that passed all the tests were mixed in a cellular composition derived from the final umbilical cord, this solution was cryopreserved for later use. When the composition was used, the cells were expanded to passage 6 using Alpha-MEM supplemented with 5% human platelet lysate (hPL).

### Example 2. Patients with decreased ejection volume

Any of the compositions described above can be used to treat any of the patients described below. The inventors have developed an effective therapeutic and / or preventive treatment for a specific subgroup of patients.

In a preferred embodiment, the patient suffers from chronic heart failure with a decreased ejection volume of the ventricular cavity.

### Inclusion criteria

In a preferred embodiment, the patient suffers from chronic heart failure with a reduced dilated ejection fraction with systolic ventricular dysfunction defined by a left ventricular ejection fraction of 40% or less by an echocardiographic evaluation, as shown in Table 1.

**Table 1 Baseline characteristics of patients with decreased ejection volume.**

| Characteristics | **Placebo (n = 15)** | **UC-MSC (n = 15)** | **p** |
|---|---|---|---|
| Age | 57.20±11.64 | 57.33±10.05 | NS |
| Gender (Men %) | 14 (93.3) | 12 (80.0) | NS |
| Ischemic heart disease (%) | 11 (73.3) | 10 (66.7) | NS |
| Arterial hypertension (%) | 8 (53.3) | 7 (46.7) | NS |
| Diabetes (%) | 7 (46.7) | 5 (33.3) | NS |
| Dyslipidemia (%) | 6 (40.0) | 7 (46.7) | NS |
| Smoker (%) | 4 (26.7) | 7 (46.7) | NS |
| Obesity (%) | 8 (53.3) | 6 (40.0) | NS |
| BMI | 29.52±4.00 | 29.12±2.88 | NS |

| Medication | | | |
|---|---|---|---|
| Aspirin (%) | 9 (60.0) | 14 (93.3) | 0.031 |
| Clopidogrel (%) | 1 (6.7) | 3 (20.0) | NS |
| Acenocoumarol (%) | 9 (60.0) | 2 (13.3) | 0.008 |
| ACEI or ARB (%) | 15 (100) | 15 (100) | NS |
| Beta blockers (%) | 15 (100) | 15 (100) | NS |
| Spironolactone (%) | 13 (86.7) | 13 (86.7) | NS |
| Other vasodilators | 2 (13.3) | 1 (6.7) | NS |
| Digitalis (%) | 1 (6.7) | 4 (26.7) | NS |
| Other antiarrhythmics (%) | 2 (13.3) | 1 (6.7) | |
| Diuretics (%) | 10 (66.7) | 9 (60.0) | NS |
| Metformin (%) | 7 (46.7) | 4 (26.7) | NS |
| Other oral antidiabetics (%) | 2 (13.3) | 0 (0.0) | NS |
| Insulin (%) | 1 (6.7) | 2 (13.3) | NS |
| Statins (%) | 12 (80.0) | 11 (73.3) | NS |
| NYHA class | 1.67±0.49 | 2.03±0.61 | NS |

| Laboratory | | | |
|---|---|---|---|
| GFR (mL / min / 1.73 m²) | 76.18 ±24.36 | 81.91±15.69 | NS |
| Hemoglobin (mg / dl) | 14.33±1.13 | 14.29±1.35 | NS |
| C-reactive protein (mg / L) | 1.65±1.41 | 1.84±1.42 | NS |
| Brain natriuretic peptide (pg / mL) | 767.45±481.02 | 451.61±495.14 | 0.015 |

| Electrocardiogram | | | |
|---|---|---|---|
| Sinus rhythm (%) | 14 (93.3) | 14 (93.3) | NS |
| LBBB (%) | 1 (6.7) | 1 (6.7) | NS |
| LAFB (%) | 3 (20.0) | 5 (33.3) | NS |
| RBBB (%) | 4 (26.7) | 2 (13.3) | NS |

| Echocardiography | | | |
|---|---|---|---|
| LVEF (%) | 31.49±4.71 | 33.00±6.18 | NS |
| LVESV (mL) | 136.53±35.32 | 108.93±38.65 | NS |
| LVEDV (mL) | 202.07±45.79 | 161.80±53.13 | 0.034 |
| Restrictive diastolic dysfunction (%) | 3 (20.0) | 1 (6.7) | NS |
| Moderate mitral regurgitation (%) | 2 (13.3) | 1 (6.7) | NS |

| | | | |
|---|---|---|---|
| BMI: body mass index. ACEI: Angiotensin converting enzyme inhibitors. ARB: Angiotensin II receptor blockers. NYHA: New York Heart Association. GFR: glomerular filtration rate according to the CKD-EPI formula. LBBB: Left bundle branch block. LAFB: Left anterior fascicular block. RBBB: Right bundle branch block. LVEF: Left ventricular ejection fraction. LVESV: Final systolic volume of the left ventricle. LVEDV Final diastolic volume of the left ventricle. NS: p> 0.05 between groups. | | | |

In a preferred embodiment, the patient should not suffer from terminal heart failure with reduced ejection fraction. In an alternative embodiment, the patient should not suffer from recurrent myocardial ischemia. In an alternative embodiment, the patient does not suffer from uncontrolled ventricular tachycardia. In an alternative embodiment, the patient should not suffer a malignant disease, in which the patient has a life expectancy of less than 1 year. In an alternative embodiment, the patient should not suffer from manifested ventricular asynchrony. In an alternative embodiment, the patient should not suffer from hematological disease. In an alternative embodiment, the patient should not suffer from a recent cerebrovascular disease, in which the disease has occurred in the last 3 months. In an alternative embodiment, the patient must have a serum creatinine level of 200 µM or less. In an alternative embodiment, the patient should not suffer from atrial fibrillation, in which the patient has not had optimal control of heart rate in the last 3 months.

In a preferred embodiment, the patient should not suffer from any of the ailments selected from the group consisting of: terminal heart failure with reduced ejection fraction, recurrent myocardial ischemia, uncontrolled ventricular tachycardia, malignant disease (in which the patient has a hope of life of less than 1 year), manifested ventricular asynchrony, hematological disease, recent cerebrovascular disease (in which the disease occurred in the last 3 months) and atrial fibrillation (in which the patient has not had optimal frequency control cardiac in the last 3 months).

In a preferred embodiment, the patient should have a serum creatinine level of 200 µM or less. In a preferred embodiment, the patient should not suffer from congestive heart failure. In a preferred embodiment, the patient must be male.

### Administration

Any of the compositions described above can be administered to any of the patients described above through any of the means described below.

The composition can be administered through any known method. For example, the composition can be administered intravenously, intralesionally, intravascularly, intraarterially, subcutaneously, intracerebrally, intramuscularly, intraperitoneally or intraventricularly. In a preferred embodiment, the composition is administered intravenously, intraarterially or intraventricularly. More preferably, the composition is administered intravenously.

Any amount of cells can be administered to a patient as long as the dosage comprises a therapeutically effective amount. In a preferred embodiment, at least 1x10⁵ cells per kilogram (kg) of the patient's body weight are administered. Preferably, at least 1x10⁵, 2x10⁵, 3x10⁵, 4x10⁵, 5x10⁵, 6x10⁵, 7x10⁵, 8x10⁵ or 9x10⁵ cells are administered per kg of the patient's body weight. More preferably, approximately 1x10⁶ cells are administered per kg of the patient's body weight. The term "approximately" refers to a variability of ± 10%.

Hydrocortisone can be administered in order to prevent any type of allergic reaction. In a preferred embodiment, the patient is administered at least 50 mg of hydrocortisone before administration of the composition. Preferably, at least 60, 70, 80 or 90 mg of hydrocortisone is administered to the patient. More preferably, 100 mg of hydrocortisone is administered to the patient before administration of the composition.

Chlorphenamine can be administered to prevent post transfusion reactions. In a preferred embodiment, the patient is administered at least 5 mg of chlorphenamine before administration of the composition. Preferably, at least 6, 7, 8 or 9 mg of chlorphenamine is administered to the patient. More preferably, 10 mg of chlorphenamine is administered to the patient before administration of the composition.

In a preferred embodiment, the patient is administered at least 50 mg of hydrocortisone and / or at least 5 mg of chlorphenamine before administration of the composition and the composition is administered at least 10 minutes after administration of hydrocortisone and / or Chlorphenamine Preferably, the composition is administered at least 15, 20 or 25 minutes after the administration of hydrocortisone and / or chlorphenamine. More preferably, the composition is administered at least 30 minutes after the administration of hydrocortisone and / or chlorphenamine.

In a preferred embodiment, 100 mg of hydrocortisone and 10 mg of chlorphenamine are administered to the patient before administration of the composition and the composition is administered at least 30 minutes after administration of hydrocortisone and / or chlorphenamine. In a preferred embodiment, hydrocortisone and / or chlorphenamine are administered once at the beginning of treatment to avoid any infusion reaction.

In a preferred embodiment, the composition is used in a combination therapy with one or more therapeutic agents selected from the group consisting of angiotensin converting enzyme inhibitors, angiotensin II receptor blockers, beta blockers, diuretics, antagonists. aldosterone, inotropic and digoxin and / or in combination with a surgical intervention or medical device selected from the group consisting of coronary bypass surgery, repair or replacement of heart valve, implantable cardioverter defibrillator, cardiac resynchronization therapy, bi-ventricular stimulation, pumps Heart and heart transplants.

Examples of angiotensin converting enzyme inhibitors include benazepril, captopril, enalapril, fosinopril, lisinopril, moexipril, perindopril, quinapril, ramipril, cilazapril, delapril, perindopril, moexipril, spirapril, temocapril, imilapril and trandolapril.

Examples of angiotensin II receptor blockers include losartan, candesartan, telmisartan, olmesartan, valsartan, irbesartan, eprosartan, tatosartan and azilesartan.

Examples of beta blockers include acebutolol, atenolol, betaxolol, bisoprolol fumarate, carteolol, carvedilol, esmolol, labetalol, metoprolol in the form tartrate and succinate, nadolol, nebivolol, penbutolol, pindolol, propanolol, sotalol, levobunolol, metolololol , Celiprolol, bucindolol and timolol.

Examples of diuretics include chlorothiazide, chlorthalidone, indapamide, hydrochlorothiazide, methiclotiazide, metolazone, bumetanide, furosemide, ethacrynate, torsemide, amiloride hydrochloride and triamterene.

Examples of aldosterone antagonists include spironolactone, eplerenone, inspra, canrenone, potassium canrenoate and spironolactone.

Examples of inotropic drugs include dobutamine, isoprenaline, enoximone, milrinone, amrinone, prenalterol, dopamine, levosimendan, digoxin, norepinephrine, adrenaline, ephedrine, methoxamine, phenylephrine, ibopamine, dopexamine, glucagon, calcium, salbutamol, pirbuterol.

In a preferred embodiment, the composition should be administered one and / or may be administered more than once. In a preferred embodiment, the patient receives a therapy regimen in which the patient is administered with a dose at the beginning of the treatment. In a preferred embodiment, the treatment should last no more than 12 months.

### Expected results of the study

Expected safety results include immediate adverse events after intravenous infusion of cellular composition derived from the umbilical cord or placebo; Incidence of global death, major cardiovascular events (defined by the combined outcome of cardiovascular deaths, hospital admission due to decompensated heart failure (HF), nonfatal myocardial infarction) and other adverse events including heart attacks, sustained ventricular arrhythmias and malignant incidents.

The expected primary assessment criterion of efficacy is a change in the left ventricular ejection fraction (LVEF) on echocardiography. In addition, an absolute 5% increase in LVEF evaluated by echocardiography was considered a clinically significant response to therapy. Secondary efficacy assessment criteria include changes in the final systolic volume of the left ventricle (LVESV) and the final diastolic volume (LVEDV) on echocardiography;

LVEF, LVESV and LVEDV in cardiac magnetic resonance imaging (CMR); Functional classification of the New York Heart Association (NYHA); General scores of the quality of life questionnaires; Maximum oxygen consumption peak (VO2 peak) and ventilatory efficiency (VE / VCO2 pending) evaluated by the cardiopulmonary exercise test; Cerebral natriuretic peptide (BNP) and high sensitivity C-reactive protein (HSCRP).

### Example 2.1: Study design and patient population

The trial was a phase 1/2, randomized, double-blind, placebo-controlled clinical trial. The study was carried out in Clínica Santa Maria and Clínica Dávila, Chile. Participants were referred from these private health centers or public hospitals and were randomly assigned between December 2012 and June 2014. The experimental design was approved by the ethics committee at the participating health centers and the Metropolitan Health Service of Chile. Prior to enrollment, all patients agreed to participate and signed an informed consent approved by the institutional review board. This study was registered at Clinicaltrials.gov (NCT01739777).

Eligible patients were enrolled in a 1:1 randomization to intravenous infusion of a cellular composition derived from the umbilical cord or placebo. The randomization list was generated with a computer by a person not related to the study. All patients were evaluated at baseline and at the pre-established follow-up points of 3, 6 and 12 months. These evaluations consisted of a clinical evaluation of adverse events and functional classification of the NYHA; Minnesota Living with Heart Failure Questionnaire (MLHFQ) and Kansas City Cardiomyopathy Questionnaire (KCCQ); laboratory analysis including complete blood count, liver and kidney function tests, cerebral natriuretic peptide (BNP) and high sensitivity C-reactive protein (HSCRP); ECG at rest, average signal ECG, 24-hour Holter ECG monitoring; Transthoracic echocardiography, cardiac magnetic resonance imaging (CMR) and cardiopulmonary exercise test. Clinical researchers, study nurses and patients were blinded to treatment allocation.

The baseline characteristics of the cellular composition derived from the umbilical cord and placebo patients did not differ in terms of demographic variables, cardiovascular risk factors, NYHA class and electrocardiography (Table 1). Ischemic cardiomyopathy was the predominant etiology of HFrEF (21 patients, 70%). There were no differences between the groups in terms of therapeutic agents that modify cardiac remodeling. No patient had implantable cardiac electronic devices. One patient from each group had left the branch block
of the beam, although none presented manifest ventricular asynchrony at the beginning. Patients treated with placebo had higher levels of BNP and a 25% higher LVEDV at baseline (p <0.05).

### Example 2.2 Preparation, characterization and infusion of the cellular composition derived from the umbilical cord.

Therapy based on a cellular composition derived from the umbilical cord was developed under good manufacturing practices (GMP) under conditions according to the FDA Guide, "Guidance for Industry: Current Good Tissue Practice (CGTP) and Additional Requirements for Manufacturers of Human Cells, Tissues, and Cellular and Tissue-Based Products (HCT / Ps)." Umbilical cords were obtained from human placentas by caesarean section, after the informed consent of healthy donors. Umbilical cords were processed as the methodology described in this document in the "Detailed description of the invention" section.

### Example 2.3: Paracrine factors.

To compare the levels of growth factor secretion between BM-MSCs and the cellular composition derived from the umbilical cord, 3X10⁴ cells were seeded in serum-free medium in 6-well plates. After 24 hours of incubation, the conditioned medium was collected and the secreted levels of vascular endothelial growth factor (VEGF) and hepatocyte growth factor (HGF) were measured using the DuoSet ELISA Development System (R&D Systems), Minneapolis, MN). In addition, the relative expression of TGFβ1 was measured using the RNeasy mini kit (Qiagen, USA) for RNA extraction and complementary DNA was synthesized in a 20 µl reaction mixture using SuperScript III First - Strand Synthesis for RT - PCR (Invitrogen, USA). RT-qPCR was performed using SYBR Green Reagents (QPCR Master Mix, Agilent Technologies). All sets of splitters were previously selected for efficacy and their sequences were: B2M (F: 5'TTCAGTTTACTCACGTCATCC 3', R: 5' ACACGGCAGGCATACTCATC 3'), TGFβ1 (F: 5'ACAATTCCTGGCGATACCTCAGCA3', F: 5'TGCAGTGTGTCTCCTGTCTCCTGCATT ').

As shown in Figure 4, the cellular composition derived from the umbilical cord showed greater expression of the TGF-β3 gene compared to BM-MSCs (p <0.001). It should be mentioned that the cellular composition derived from the umbilical cord showed a 55 times greater expression of HGF compared to BM-MSCs (p> 0.0001) (Figure 4), which in some cases showed undetected levels of HGF. This last factor is a critical test to select which umbilical cords and / or cell populations are going to be used.

### Example 2.4 Immunomodulatory effects

The immunosuppressive properties of the cellular composition derived from the umbilical cord were analyzed by assessing its effect on the proliferative response of peripheral blood mononuclear cells (PBMCs) after an in vitro PHA stimulation, as shown in Figure 6.

Most preferably, the proliferation of different T-cell subgroups of 4 patients with HFrEF included in this assay was performed in vitro to assess the immunomodulatory effect of the cellular composition derived from the umbilical cord and BM-MSC. Mononuclear cells were isolated from human peripheral blood by a density gradient of Ficoll-Paque Plus (GE Healthcare, Amersham, RU) (1,077 g / ml) according to the manufacturer's instructions.

PBMC cells were stained with carboxyfluorescein succinimidyl ester (CFSE, Life Technologies, Carlsbad, CA) following the manufacturer's protocol, co-cultured with MSC in 96-well plates in a 1:10 ratio (MSCs: hPBMC) in medium RPMI (Roswell Park Memorial Institute medium) (Sigma-Aldrich, USA) supplemented with 10% FBS, 1% LG, 1% non-essential amino acid (sigma-Aldrich, USA), 100mM sodium pyruvate (sigma, USA), 25mMb -mercaptoethanol (Gibco, NY) and 15mg / ml of phytohemagglutinin (PHA) (Sigma, USA).

After 72 hours, PBMCs were stimulated for 4 hours with 50 ng / ml formbolmisteterottate (PMA) (Sigma Aldrich) and 1 µg / ml ionomycin (Sigma-Aldrich) in the presence of Brefeldin A (Biolegend, San Diego, CA,).

For surface staining, cells were incubated with antibodies against human CD4, CD8, CD3 and CD25 (BD Biosciences, USA) in the dark at 4 ° C for 30 min. Intracellular staining was performed using BD Cytofix / Cytoperm solution, according to the manufacturer's protocol with antibodies against human IL-17, IL4 and IFNγ (eBioscience, USA). For the evaluation of the transcriptional factor, FoxP3 expression was evaluated with staining buffer and specific antibodies (eBioscience, USA) according to the manufacturer's protocol. The cells were acquired using a FACS Canto II Flow cytometer (BD Biosciences) and analyzed with FlowJo software (Tristar, Stanford) for phenotype and proliferation, calculated by the decrease in CFSE fluorescence, as shown in Figure 6.

The cellular composition derived from the umbilical cord showed a similar inhibitory effect on T cell proliferation compared to BM-MSCs in the 1:10 ratio, the percentages of inhibition were 21.53% ± 3.85% and 23.96% ± 4.50% for UC-MSCs and BM-MSCs with respect to

PHA-induced proliferation in the absence of MSC (p <0.005 vs. control) (Figure 6A). Collaborating T cells 1, collaborating T cells 2 and cytotoxic T cells showed a tendency to decrease their proliferation after being co-cultured with the cellular composition derived from the umbilical cord or BM-MSCs (p> 0.05). No effect of co-cultures of the umbilical cord derived cell composition on the proliferation of regulatory T cells was observed (Figure 6B).

### Example 2.5 Migration Profile

For the migration experiment, cell migration assays were performed with the Transwell two chamber cell culture method (Corning, Cambridge, MA) with an 8 µm pore polycarbonate membrane, as shown in Figure 7. The upper side of the Transwell membrane was coated with 0.1% gelatin in PBS (Sigma-Aldrich, St. Louis, MO) for 2 h at 37 ° C. The cellular composition derived from the umbilical cord and BM-MSCs were seeded separately at a density of 15,000 cells per 100 µl of 1% DMEM P / S, 0.1% FBS in the upper chamber of the Transwell apparatus. The cells were allowed to migrate to the medium (500 µl) in the lower chamber containing DMEM alone or supplemented with 5% serum isolated from patients with HFrEF. The Transwell system was incubated for 16 h at 37 ° C in a humidified atmosphere containing 5% CO2. After incubation, the non-migratory cells were carefully removed from the upper face of the Transwell insert with a cotton swab. The bound cells remaining in the Transwell insert were fixed with 70% methanol and stained with 1% violet crystal in 20% methanol for 1 h. After washing, the stained cells that migrated from the top to the bottom of the membrane were counted under a bright field inverted microscope with a 20X magnification. The number of migrated cells was expressed as the percentage change of the control value (DMEM only). Each experiment was performed in biological and experimental triplicate. The percentage of migratory cells was significantly higher in the cellular composition derived from the umbilical cord compared to BM-MSCs in response to the serum of the HFrEF patient (41.18 ± 6.53 vs. 29.67 ± 8.35; p <0 , 01), as shown in Figure 7. Validating thus, that the cells obtained by the methodology presented in this invention have a high migration.

### Example 2.6: Security

The humoral immune response to the infusion of a cellular composition derived from the allogeneic umbilical cord was evaluated in a group of 12 patients (7 treated with UC-MSC, 5 who received placebo) at days 0, 15 and 90 of infusion of therapy using Luminex 200 (Kashi Clinical Laboratories Inc Portland).

There were no acute adverse events associated with the infusion of allogeneic cellular composition derived from the umbilical cord or placebo. None of the individuals evaluated developed antibodies against alloantigens after infusion. It is noteworthy that a patient with baseline reactivity at 52 different HLA specificities before the infusion of the umbilical cord derived cellular composition lost reactivity to 16 of these specificities at day 90. In addition, since the infused cells were typed, it was possible detect that only 21% of the specificities not expressed in the umbilical cord administered cellular composition disappeared, as opposed to 100% of the specificities present in the umbilical cord administered cellular composition (p = 0.004). The results not only confirm the absence of humoral immune reaction to the cellular composition derived from the umbilical cord, but also confirm that MSC preferentially suppresses reactivity to its own HLA molecules, as is known from the literature.

### Example 2.7: Clinically relevant events

Clinically relevant events throughout the 12-month follow-up are shown in Table 2. The deceased patient of the placebo group had an acute myocardial infarction at 5 months of follow-up. A patient of the umbilical cord derived cellular composition developed acute lymphocytic leukemia 5 months after intravenous infusion of the umbilical cord derived cellular composition, lacking clinical and laboratory elements suggestive of leukemia at baseline and at 3 months follow-up. A patient in the placebo group developed a malignant melanoma. Regarding major cardiovascular events, three patients of the placebo group and one of the UC-MSC had hospitalizations due to decompensation of their heart failure, only one patient experienced an acute coronary syndrome in the placebo group. None of the patients presented an acute ischemic stroke. Newly occurring supraventricular arrhythmias, sustained ventricular arrhythmias, atrioventricular blockages or bundle branch block during the follow-up were not diagnosed, and none were observed in the Holter ECG monitoring. There was an increase in the amount of premature ventricular complexes in 24-hour ECG monitoring in the placebo group at follow-up, although without changes in the mean of the Lown classification. No significant variations were observed in the time domains or frequency in the follow-up. No thoracic ectopic tissue formation was observed in CMR upon completion of this study. No significant abnormalities were observed in complete blood counts, renal and hepatic function during monitoring points.

**Table 2 Incidence of clinically relevant events at 12 months follow-up.**

| | Placebo (n = 15) | UC-MSC (n = 15) | P |
|---|---|---|---|
| General deaths | 1 | 1 | NS |
| Cardiovascular deaths | 1 | 0 | NS |
| Hospitalizations | 4 | 1 | NS |
| Heart failure | 3 | 1 | NS |
| Myocardial infarction | 1 | 0 | NS |
| Malignant incident | 1 | 1 | NS |
| Ventricular Tachycardia Not Sustained | 7 | 7 | NS |

| | | | |
|---|---|---|---|
| NS: p>0.05 between groups. | | | |

### Example 2.8: Cardiac Image

The echocardiographic parameters evaluated at baseline and follow-up are shown in Table 3. Compared to baseline, there were improvements in LVEF in the group treated with the cellular composition derived from the umbilical cord that began at 3 follow-up months (+3.71 ± 5.01% p = 0.010), and continued at 6 months (+ 5.43 ± 4.99%, p = 0.001) and 12 months (+ 7.07 ± 6, 22%, p = 0.001). There were no changes in the volumes of the left ventricle. The placebo group did not show large differences in these variables. The change in LVEF from baseline to month 12 differed significantly for both groups (+ 7.07 ± 6.22% vs + 1.85 ± 5.60%, p = 0.028). Regarding the response to treatment, 11 patients in the cell composition group derived from the umbilical cord and 3 patients in the placebo group presented a clinically relevant improvement in the LVEF (78.6% versus 35.7%; p = 0.054). Responders in the umbilical cord-derived cell composition group had an average increase of + 6.73 ± 4.82% at 6 months (p = 0.005 compared to baseline) and + 9.18 ± 5.15 % at 12 months (p = 0.002 compared to baseline). In addition, 7 out of 14 patients presented a significant increase in LVEF and an LVEF> 40% at 12 months after administration of the cellular composition derived from the umbilical cord, while only 1 of the placebo group met both conditions (50, 0% vs. 7.1% p = 0.0365).

The CMR measurements are shown in Table 3. Patients treated with intravenous infusion of the cellular composition derived from the umbilical cord presented an increase in LVEF (p = 0.0003) and LVEDV (p = 0.012) (Figure 8). The most significant improvement of the LVEF was at 6 months follow-up (+ 4.67 ± 4.51, p = 0.005). There was an increase in LVEDV treated with the cellular composition derived from the umbilical cord at 12 months (p = 0.033). No changes were observed in LVEF or left ventricular volumes in the placebo group (n = 13). A patient in the placebo group withdrew consent for CMR.

**Table 3 Results of primary and secondary efficacy at baseline and follow-up points**

| *Variable* | *Group* | *n* | *Baseline* | *3 months* | *6 months* | *12 months* |
|---|---|---|---|---|---|---|
| **TTE LVEF** | **Placeb o** | 1 4 | 31.53±4.89 | 33.00±7.24 | 32.79±7.76 | 33.39±7.38 |
| | **UC-MSC** | 1 4 | 33.50±6.09 | 37.21±6.80‡ | 38.93±5.74‡ | 40.57±8.19‡ |
| **TTE LVESV** | **Placeb o** | 1 4 | 134.3±35.5 | 126.4±39.8 | 128.4±43.4 | 131.1±42.0 |
| | **UC-MSC** | 1 4 | 110.1±37.6 | 99.1±39.0 | 104.4±42.8 | 100.5±36.8 |
| **TTE LVEDV** | **Placeb o** | 1 4 | 199.2±46.1 | 188.8±42.1† | 189.1±45.4 | 191.7±43.5 |
| | **UC-MSC** | 1 4 | 168.1±48.5 | 161.6±43.9 | 167.0±56.6 | 161.4±48.6 |
| **CMR LVEF** | **Placeb o** | 1 3 | 29.62±6.53 | 28.80±6.55 | 30.66±7.65 | 31.31±7.10 |
| | **UC-MSC** | 1 4 | 32.64±8.42 | 35.93±9.83† | 38.41±12.00 ‡ | 37.43±10.44 † |
| **CMR LVESV** | **Placeb o** | 1 3 | 175.2±56.8 | 170.9±39.8 | 167.8±50.5 | 179.0±52.6 |
| | **UC-MSC** | 1 4 | 130.2±42.8 * | 130.8±62.1 | 121.3±46.2 | 133.9±62.1 |
| **CMR LVEDV** | **Placeb o** | 1 3 | 245.9±60.1 | 207.3±75.4 | 241.0±56.0 | 257.8±54.1 |
| | **UC-MSC** | 1 4 | 185.5±50.0 * | 197.7±67.2 | 190.8±48.2 | 210.0±67.2† |
| **NYHA** | **Placeb o** | 1 4 | 1.71±0.48 | 1.50±0.62 | 1.43±0.55t | 1.46±0.63 |
| | **UC-MSC** | 1 4 | 2.07±0.62 | 1.57±0.61‡ | 1.50±0.59‡ | 1.43±0.63‡ |
| **MLHFQ** | **Placeb o** | 1 4 | 37.42±22.2 2 | 29.04±18.39 | 26.86±22.93 | 27.07±20.36 |
| | **UC-MSC** | 1 4 | 53.21±30.2 5 | 30.50±23.76 † | 27.07±21.54 ‡ | 31.21±26.66 † |
| **KCCQ-CS** | **Placeb o** | 1 4 | 69.92±21.2 4 | 78.08±15.94 † | 78.64±18.46 † | 75.46±22.43 |
| | **UC-MSC** | 1 4 | 57.48±25.3 3 | 73.22±22.89 † | 74.99±20.70 † | 72.82±24.10 ‡ |
| **VO2 peak** | **Placeb o** | 1 4 | 17.56±5.04 | 18.14±5.32 | 17.85±4.92 | 18.16±4.70 |
| | **UC-MSC** | 1 4 | 18.11±4.67 | 18.52±4.28 | 18.59±4.84 | 17.88±4.11 |
| **VE/VCO 2** | **Placeb o** | 1 4 | 34.42±5.12 | 34.19±6.01 | 33.61±6.28 | 33.42±6.74 |
| | **UC-MSC** | 1 4 | 34.06±8.53 | 32.11±5.99 | 32.41±5.18 | 32.17±7.41† |
| **BNP** | **Placeb o** | 1 4 | 731±477 | 654±468 | 681±499 | 892±801 |
| | **UC-MSC** | 1 4 | 474±507* | 355±443‡ | 452±586 | 394±535† |
| **HSCRP** | **Placeb o** | 1 4 | 1.63±1.46 | 1.78±1.86 | 1.92±1.85 | 1.67±1.09 |
| | **UC-MSC** | 1 4 | 1.68±1.33 | 3.15±3.60 | 5.15±15.94 | 2.15±2.58 |

| | | | | | | |
|---|---|---|---|---|---|---|
| TTE: transthoracic echocardiogram. CMR: Cardiac Magnetic Resonance. LVEF: Left ventricular ejection fraction (%). LVESV: final systolic volume of the left ventricle (ml). LVEDV: Final diastolic volume of the left ventricle (ml). NYHA: New York Heart Association. MLHFQ: "Minnesota Living with Heart Failure" questionnaire. KCCQ-CS: Questionnaire "Kansas City Cardiomyopathy Questionnaire Clinical Summary". VO2 peak: Maximum oxygen consumption (ml / Kg / min). VE / VCO2: Minimum ventilation speed for the carbon dioxide production ratio. BNP: brain natriuretic peptide (pg / ml). HSCRP: High sensitivity C-reactive protein (mg / L). * P <0.05 versus placebo. † p <0.05 versus baseline condition. ‡ p <0.0167 against the baseline condition. | | | | | | |

### Example 2.9: Functional status, quality of life and clinical biomarkers

The results are summarized in Table 3. There were substantial improvements in the NYHA class in patients treated with the cellular composition derived from the umbilical cord, beginning at 3 months (-0.54 ± 0.56; p = 0.011), which remained at 12 months follow-up (- 0.62 ± 0.46, p = 0.003). Only the group treated with the cellular composition derived from the umbilical cord experienced improvements in MLHFQ from baseline to all follow-up points (p <0.05). Both groups experienced an initial improvement of the clinical summary of the KCCQ at 3 and 6 months of follow-up, with persistence of improvement at the end of the trial only in the group treated with the cellular composition derived from the umbilical cord (p = 0.014). Patients treated with the cellular composition derived from the umbilical cord showed an improvement in VE / VCO₂ at 12 months (-1.89 ± 3.19, p = 0.023 versus baseline), while no differences were observed in the peak of VO₂. No differences were found in other variables of exercise capacity, including METS, oxygen consumption at the anaerobic threshold, maximum respiratory exchange ratio and exercise time after cell therapy, as shown in Table 3. A Slight decrease in BNP levels in the group treated with the cellular composition derived from the umbilical cord at 3 and 12 months of follow-up.

### Conclusions

Intravenous infusion of the subpopulation of cells obtained through this invention was feasible and safe in this group of patients with HFrEF under optimal medical treatment. This allogeneic treatment did not induce humoral immune response in tested individuals. The intervention resulted in a significant improvement in left ventricular function, functional status and quality of life. Thus validating, the use of the solution of cellular composition derived from the umbilical cord described in this invention for this application.

### Example 3. Patients with problems in the interaction between the femur, the patella and the tibia.

Any of the compositions described above can be used to treat any patient described below. The inventors have developed an effective therapeutic and / or preventive treatment for a specific subgroup of patients.

In a preferred embodiment, the patient suffers problems of pain and functionality in the knee. Preferably, the patient suffers from problems of pain and functionality in the knee due to the wear generated in the interaction between the femur, the patella and the tibia.

### Inclusion criteria

In a preferred embodiment, the patient should suffer from symptomatic knee osteoarthritis (OA). More preferably, the patient should suffer from daily pain in the affected joint within 12 months before inclusion.

As shown in Table 4, the patient must be between 40-46 years of age. In an alternate embodiment, the patient must have radiographic changes of grade I, II or III according to the Kellgren-Lawrence scale and the stable target knee.

In an alternative embodiment, the patient should not suffer symptomatic bilateral knee OA. In an alternative embodiment, the patient should not suffer from generalized bone marrow edema with condylar or tibial plateau in MRI. In an alternative embodiment, the patient should not have a knee deviation of axial deviation greater than 10 ° defined by Valgus, and / or a knee deformation greater than 5 ° according to Varus. In an alternative embodiment, the patient should not have used oral or intravenous administration of steroids or hyaluronic acid in the last 6 months before the start of treatment. In an alternative embodiment, the patient should not have ipsilateral hip or ankle pain. In an alternative embodiment, the patient should not have any local or systemic infection. In an alternative embodiment, the patient should not have any form of secondary arthritis. In an alternative embodiment, the patient should not have any previous malignancy.

**Table 4 Baseline characteristics for patients suffering from pain and functional problems in the knee.**

| | **HA group (HA, n=9)** | **MSC single dose group** (UC-MSC 20x106 at basekine, n=10) | **MSC sequential dose group** (UC-MSC 20x106 at basekine, and 24 weeks, n=10) | **P Value** |
|---|---|---|---|---|
| **Age, yr** | 54.7 ± 1.6 | 55.4 ± 2.3 | 57.8 ± 1.3 | 0.76 |
| **Female, %** | 50 | 44 | 55 | 0.89 |
| **BMI, kg/m2** | 27.8 | 27.6 | 27.4 | 0.95 |
| **Kellgren grade (%)** | | | | 0.69 |
| II | 75 | 55 | 66 | |
| III | 25 | 45 | 33 | |

| **WOMAC INDEX** | | | | |
|---|---|---|---|---|
| Total | 29 ± 4.7 | 37.4± 4.2 | 34.6±3.1 | 0.04 |
| A Pain (0-20= | 7.0± 0.9 | 9.2± 1.2 | 8.4± 0.6 | 0.27 |
| B Stiffness (0-8) | 3.2± 0.7 | 2.8± 0.4 | 2.8± 0.5 | 0.89 |
| C Function (0-68) | 18.7± 3.9 | 25.3± 3.0 | 23.7± 3.1 | 0.35 |
| **Global knee pain, Vas 0-100 (mm)** | 38.7± 6.8 | 44.7± 5.8 | 40.1± 7.6 | 0.15 |

| **SF-36** | | | | |
|---|---|---|---|---|
| Physical scale | 51.3± 7.4 | 46.9± 5.8 | 60± 6.3 | 0.21 |
| Pain scale | 48.4± 6.8 | 52.2± 7.8 | 56.6± 7.0 | 0.59 |
| **Health status scale** | 66.9± 8.0 | 70± 4.7 | 74.4± 7.5 | 0.74 |
| **WORMS, 0-332 points** | 30.9± 9.5 | 46.1 ± 7.3 | 40.1 ± 7.9 | 0.67 |

### Administration

Any of the compositions described above can be administered to any of the patients described above through any of the means described below.

The composition can be administered through any known method. For example, the composition can be administered intravenously, intralesionally, intravascularly, intraarterially, subcutaneously, intracerebrally, intramuscularly, intraperitoneally or intraventricularly. In a preferred embodiment, the composition is administered intralesionally, subcutaneously or intraarticularly. Preferably, the composition is administered intra-articular.

Any amount of cells can be administered to a patient as long as the dosage comprises a therapeutically effective amount. In a preferred embodiment, at least 1x10⁶ cells are administered per patient. Preferably, at least 5 x 10⁶, 10 x 10⁶ or 15 x 10⁶ cells are administered per patient. More preferably, at least about 20x10⁶ cells are administered per patient. The term "approximately" refers to a variability of ± 10%.

Most preferably, a total of 20x10⁶ cells suspended in a final volume of 3 ml (saline solution, 5% AB plasma) and dispensed in 5 ml syringes, in one or more specified doses according to the study design will be injected intraarticularly.

The number of doses administered to the patient must be related to the duration of the treatment. In a preferred embodiment, at least one dose should be administered for treatment, at the beginning of treatment, this treatment should not last more than 52 weeks. Preferably, at least two doses should be administered for treatment, one at the beginning and one in the middle of the treatment, for a treatment that does not last more than 52 weeks. More preferably, at least two doses will be administered for treatment, one at the beginning and one at week 24 of the treatment, for a treatment that does not last more than 52 weeks.

### Expected results of the study

The primary assessment criterion of the trial was safety. Adverse events (EA) were coded by the classification of Common Terminology Criteria for Adverse Events (CTCAE). AE were documented at each visit and described in terms of severity, incidence and relationship with intra-articular infiltration.

The secondary objective of the trial was efficacy. The following clinical endpoints Western Ontario and Mc Master Universities Arthritis Index (WOMAC), visual analog scale of the pain (VAS), quality of life through the Short-form 36 (SF-36) questionnaire, Patient Global Assessment (PGA) and OMERACT-OARSI Respondent Index Criteria. For the structural evaluation, an MRI was performed at baseline and later at 6 and 12 months by an impartial radiologist. The whole organ magnetic resonance imaging (WORMS) score was used.

### Example 3.1: Study design and patient population

This trial corresponded to a randomized, double-blind, controlled comparator, active comparator against cellular composition derived from the allogeneic umbilical cord, phase I / II study that evaluated the safety and efficacy of intra-articular injection of a cellular composition derived from the umbilical cord in OA of the knee, as can be seen in Figure 9. The patients provided their written informed consent before the clinical study. All subjects were recruited between November 2015 and January 2016 at the Clinical Center of the Universidad de los Andes, Chile. Forty patients were selected and 29 patients were assigned consecutively to the treatment groups: The MSC-1 group received a dose of a cellular composition derived from the umbilical cord in an injection at the start of the study. The MSC-2 group received a sequential dose of a cellular composition derived from the umbilical cord at the start of the study and 6 months later. The control group (n = 9) received hyaluronic acid (HA) at the start of the study and 6 months later. An electronic data entry system was used for randomization. All data were masked for people who perform the procedures and the evaluation of the clinical data. One patient from each group dropped out of the study before the administration of the treatment (Figure 8). The study was registered at ClinicalTrials.gov (NCT02580695) and was carried out in accordance with the guidelines of good clinical practice (GCP) and the Declaration of Helsinki. The approval was obtained from the regional ethical committee of the Ministry of Health of Chile (CECSSMO131015).

### Example 3.2 Preparation, characterization and infusion of the cellular composition derived from the umbilical cord

The manufacture of the cellular composition derived from the umbilical cord was approved by the Ethical Committee of the Dávila Clinic and the Eastern Metropolitan Health Service (SSMO). Therapy based on a cellular composition derived from the umbilical cord was developed under good manufacturing practices (GMP) under conditions according to the FDA Guide, "Guidance for Industry: Current Good Tissue Practice (CGTP) and Additional Requirements for Manufacturers of Human Cells, Tissues, and Cellular and Tissue-Based Products (HCT / Ps)". Umbilical cords were obtained from human placentas by caesarean section, after an informed consent of healthy donors. Umbilical cords were processed as the methodology described in this document in the "Detailed description of the invention" section.

### Example 3.3: Security

No serious adverse events were recorded during the trial. Regarding AS related to intra-articular injection, no infectious events occurred. The effusion evaluation was medium to moderate in the first week after the injection, it was higher in the MSCs groups without reaching statistical significance (1st injection, 34% MSC1, 33% MSC2 vs. 25% HA, p = 0.64 and 2nd injection MSC2 41% vs. HA 37.5%, P = 0.76), as shown in Table 5.

**Table 5 Safety of intra-articular injection of a cellular composition derived from the umbilical cord in the knee with OA**

| | **HA group** | **MSC single dose group** | **MSC sequential dose group** | **P Value** |
|---|---|---|---|---|
| **Postransplantation AE, % (2nd injection)** | | | | |
| Septic arthritis | 0 (0) | 0 (0) | 0 (0) | - |
| Reactive effusion | 25 (37.5) | 44 (0) | 33 (44) | 0.26 |
| - Mild | 25 (37.5) | 33 (0) | 33 (33) | 0.43 |
| - Moderate | 0 (0) | 11 (0) | 0 | 0.17 |
| - Severe | 0 (0) | 0 (0) | 0 (11) | 0.17 |
| Pain | 0 (0) | 11 (0) | 11 (11) | 0.21 |
| Fever | 0 | 0 | 0 | - |
| Urticarial lesions | 0 | 0 | 0 | - |
| Bleeding | 0 | 0 | 0 | - |

| **AE during follow-Up%** | | | | |
|---|---|---|---|---|
| Fatal | 0 | 0 | 0 | - |
| Serious | 0 | 0 | 0 | - |
| Non serious | 0 | 0 | 0 | - |

### Example 3.4: Clinical Results

As for the mean change from the baseline, only the groups of cellular composition derived from the umbilical cord show a significant improvement. The comparison between three study groups showed that both groups of cellular composition derived from the umbilical cord had a statistical superiority compared to HA in pain and function at 6 months, as shown in Figure 10 and Figure 11. Additionally, patients treated twice with a cellular composition derived from umbilical cord was superior to HA in all the clinical results described in the total WOMAC evaluation presented in Figure 11 (WOMACₜₒₜₐₗ MSC2 4.44 ± 1.76 versus HA 12.6 ± 4, 5, 95% CI 5.1, 21.3, p <0.01). At the end of the follow-up, 62.5% of the patients in the HA group were responders compared to 100% of the patients in the MSC sequential dose group measured by the OMERACT-OARSI response rate criteria (p < 0.05), as shown in Figure 13.

### Example 3.5: Structural Results

For the evaluation of structural changes, no changes were observed in the 14 characteristics of the WORMS score, including meniscus, cartilage, synovitis, osteophytes and subarticular bone marrow at 12 months, as shown in Figure 14.

### Conclusions

Intra-articular injection of the cellular composition derived from the umbilical cord obtained through this invention is feasible and safe in this group of patients. This allogeneic treatment demonstrated the safety and clinical efficacy of the cellular composition derived from the umbilical cord in knee OA and provides evidence to consider this cell therapy as a therapeutic alternative for the treatment of OA. Validating thus, the use of the solution of cellular composition derived from the umbilical cord described in this invention for this application and for similar applications as in OA of Hip, ankle and wrist. More specifically, the cellular composition derived from the umbilical cord may have effects on the wear generated between the head of the femur with the acetabulum, between the talus-tibia-fibula and between radius-ulna-bones of the carpus / carpal.

As far as we know, this example is the first trial to compare two sequential doses of a cellular composition derived from umbilical cord versus a single dose of cell therapy and versus an active comparator such as hyaluronic acid.

### Example 4. Obtaining subpopulation of cells of the invention for clinical application.

To obtain the subpopulation of cells of the invention, samples of 5 different umbilical cords obtained from full-stage human placentas were taken, these placentas were extracted by caesarean section after the informed consent of a healthy donor; to subsequently be stored aseptically in a buffer solution with antibiotics, as described in detail in the "cord processing" section.

Subsequently, the cords were cross-sectioned into 8-10 cm segments following the model presented in Figure 2A, then three longitudinal cuts were made to expose and then remove the vein and umbilical arteries, as shown in Figure 2B. Positioning the internal faces surrounding the arteries and vein in contact with the bottom of the culture plates, as shown in Figure 2C. The cells that migrated and adhered to the plates were expanded with the medium detailed in the section "Isolation of the cellular subpopulation by explant".

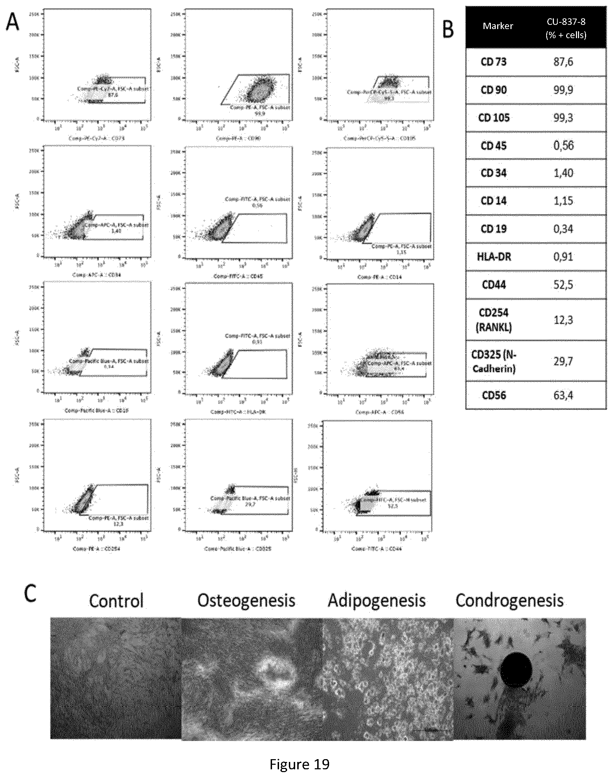

The different cell batches were validated by means of the protocol described in the section "Characterization according to the guidelines of the International Society of Cell Therapy". The results obtained for the markers CD73, CD90, CD105, CD45, CD34, CD14, CD19, HLA-DR, and in vitro differentiation in osteoblasts, adipocytes and chondroblasts, are shown in Figure 15 to 19. Table 6 shows the results.

**Table 6 Characterization of cell batches according to the guidelines of the International Society of Cell Therapy.**

| **Cell Marker** | **Criterio MSC*** | **CU-989-6** | **CU-745-3** | **CU-108-6** | **CU-668-K** | **CU-837-8** |
|---|---|---|---|---|---|---|
| | (% of cells +) | (% of cells +) | (% of cells +) | (% of cells +) | (% of cells +) | (% of cells +) |
| **CD 73** | >95% | 91,7 | 97,8 | 100 | 100 | 87,6 |
| **CD 90** | >95% | 100 | 98 | 100 | 100 | 99,9 |
| **CD 105** | >95% | 100 | 98,7 | 99,8 | 99,9 | 99,3 |
| **CD 45** | <2% | 0,59 | 0,25 | 0,09 | 0,009 | 0,56 |
| **CD 34** | <2% | 1,56 | 0,15 | 0,21 | 0,026 | 1,4 |
| **CD 14** | <2% | 1,14 | 0,03 | 0,08 | 0,058 | 1,15 |
| **CD 19** | <2% | 0,7 | 0,13 | 0,06 | 0,008 | 0,34 |
| **HLA-DR** | <2% | 1,8 | 0,58 | 0,06 | 0,036 | 0,91 |

| | | | | | | |
|---|---|---|---|---|---|---|
| **(Dominici et al. 2006).* | | | | | | |

Because the isolated cell batches of the CU-989-6 and Cu-837-8 cords have a presence of the CD73 marker less than 95%, these batches cannot be used since they do not meet the first requirement of this method of selection described in the section "Characterization according to the guidelines of the International Society of Cell Therapy" (Dominici et al. 2006. Minimal criteria for defining multipotent mesenchymal stromal cells. The International Society for cellular Therapy position statement. Cytotherapy Vol. 8, No. 4, 315-317).

The cell batches CU-745-3, CU-108-6 and CU-668-K do meet the criteria for selection of mesenchymal stem cells, so the markers described in the section "Characterization of MSC subpopulations" were analyzed, the results are found in figures 16, 17 and 18 and in Table 7.

**Table 7 Characterization of MSC subpopulations according to the selection markers described in this patent**

| | **CU-745-3** | **CU-108-6** | **CU-668-K** |
|---|---|---|---|
| **Cell Marker** | (% of cells +) | (% of cells +) | (% of cells +) |
| **CD44** | 19,5 | 79,6 | 27,3 |
| **CD56** | 55,3 | 32,5 | 60,1 |
| **CD 105** | 98,7 | 99,8 | 99,9 |
| **RANKL** | 10,2 | 79,9 | 23,6 |
| **CD44** | 19,5 | 79,6 | 27,3 |

The populations analyzed, from 3 different umbilical cords, have the markers CD44, CD56, CD105, RANKL and N-cadherin, therefore, represent the required subpopulation. **Specifically, the 3 cell cultures evaluated have low, medium and high concentrations for the CD44, CD56, RANKL and N-cadherin markers. Therefore, it is demonstrated that only the presence of the invention markers is sufficient to obtain the desired therapeutic results.**

Once the characterization of cell subpopulations was approved, the different batches were cryopreserved for later use. Specifically, such batches were used in the following examples:
- Lot CU-108-6 was used for Example 3. "Patients with problems in the interaction between the femur, patella and tibia", for Example 6. "Use of Mesenchymal Subpopulation for patients with a volume of decreased ejection"and for Example 8 "Use of Mesenchymal Subpopulation to treat skin wounds".
- Lot 745-3 was used in Example 5. "Use of Mesenchymal Subpopulation for patients with problems in the interaction between the femur, patella and tibia", for Example 6 "Use of Mesenchymal Subpopulation for patients with a decreased ejection volume" and for Example 8 "Use of Mesenchymal Subpopulation to treat skin wounds".
- Lot CU-668-K was used in Example 7. "Use of Mesenchymal Subpopulation for patients suffering from respiratory injuries".

### Example 5. Use of Mesenchymal Subpopulation for patients with osteoarthritis.

Any of the compositions approved by the selection criteria "MSC subpopulation characterization" described herein can be administered to any of the indications described herein. Specifically, the CU-745-3 cell composition selected in Example 4 "Obtaining subpopulation of cells of the invention for clinical application" was used for the treatment of patients of the present example.

The inventors have developed an effective therapeutic and / or preventive treatment for a specific group of indications.

In one embodiment, the patient to whom the treatment of the invention is applied suffers problems of pain and functionality in the knee. Treatment can be especially useful in patients suffering from pain and knee function problems due to the degenerative disorder generated in the interaction between the femur, patella and tibia.

The patient to whom the treatment of the invention is applied suffers from symptomatic osteoarthritis. The treatment can be especially useful in patients suffering from pain and functional impotence compromising one or more of the joints that are the seat of symptomatic ostearthritis.

### Inclusion criteria

Candidates for the treatment of the invention are selected from patients suffering from osteoarthritis (OA). And especially among patients who present with functionally limiting chronic pain, daily in the affected joint within 6 months before inclusion.

Patients with radiographic changes of grade I, II or III according to the Kellgren-Lawrence scale are also considered as candidates for the treatment of the invention. It is important that the patient receiving the treatment should not have any local or systemic infection. Likewise, the candidate patient should not have any form of secondary arthritis, nor any previous malignancy.

For this example, 7 patients who met the inclusion criteria indicated with the developed cell composition of lot CU-745-3 were treated, since they met the method of selection of the invention.

### Administration

Any of the compositions approved by the selection criteria "Characterization of subpopulations of MSC" described in this document, can be administered to any of the patients who meet the inclusion criteria, through any of the means available in the art, for example those described below.

The cellular composition can be administered through any known method. For example, the cellular composition can be administered intravenously, intralesionally, intraarticularly, intravascularly, intraarterially, subcutaneously, intracerebro-ventricularly, intramuscularly, intraperitoneally or intraventricular In a preferred embodiment, the composition is administered intralesionally, subcutaneously or intraarticularly. Preferably, the composition is administered intra-articular.

Any amount of cells can be administered to a patient as long as the dosage comprises a therapeutically effective amount. In a preferred embodiment, at least 1x10⁶ cells are administered per patient. Preferably, at least 5 x 10⁶, 10 x 10⁶ or 15 x 10⁶ cells are administered per patient. More preferably, at least about 20x10⁶ cells are administered per patient. The term "approximately" refers to a variability of ± 10%.

Most preferably, a total of 20x10⁶ cells suspended in a final volume of 3 ml (saline solution, 5% AB plasma) and dispensed in 5 ml syringes, in one or more specified doses according to the study design will be injected intraarticularly .

The number of doses administered to the patient must be related to the duration of the treatment. In a preferred embodiment, at least one dose should be administered for treatment, at the beginning of treatment, this treatment should not last more than 52 weeks. Preferably, at least two doses should be administered for treatment, one at the beginning and one in the middle of the treatment, for a treatment that does not last more than 52 weeks. More preferably, at least two doses will be administered for the treatment, one at the beginning and one at the middle of the treatment, for a treatment that does not last more than 52 weeks.

### Results of the treatment

To assess the efficacy of the therapy, the progress of the patients was analyzed based on the Western Ontario clinical questionnaire and the McMaster Universities Arthritis Index (WOMAC). This analysis was separated into two comparisons, the first comparison focuses on the 3-month progress of patients, these results are shown below in Figure 20.

As can be seen in Figure 20, at 3 months of treatment the administered cellular composition helps to reduce pain, stiffness and improve functionality according to clinical index validated on average more than 4.5 points according to the total WOMAC measurement. Specifically, at 3 months a decrease in pain and stiffness of 3.3 and 1.3 points respectively is seen, more notably for the case of functionality the average of the patients improves on average 10 points according to the WOMAC index.

The second comparison shows the evolution at 3 and 6 months post treatment, these results are shown below in Figure 21.

As can be seen, at 6 months the administered cellular composition also decreases pain, stiffness and improving functionality 2.3 points in relation to 3 months according to the WOMAC Total index. Specifically, at 6 months an average decrease in pain and stiffness of 2 and 1.3 points, respectively, is seen, more notably for the case of functionality the average of the patients improves on average 3.7 points.

### Therefore, it is demonstrated that the cellular composition selected by the methodology described in this document benefits patients with osteoarthritis.

### Example 6. Use of Mesenchymal Subpopulation for patients with decreased ejection volume

### Inclusion criteria

Candidates for the treatment of the invention were selected from patients suffering from chronic heart failure with a reduced ejection fraction in dilated stage with systolic ventricular dysfunction defined by a left ventricular ejection fraction of 40% or less by an echocardiographic evaluation.

For this example, 3 patients were treated with cellular compositions developed from lots CU-108-6 and CU-745-3 that comply with the method of selection of the invention.

### Administration

Any of the compositions described above can be administered to any of the patients described above through any of the means described below.

The composition can be administered through any known method. For example, the composition can be administered intravenously, intralesionally, intravascularly, intraarterially, subcutaneously, intracerebrally, intramuscularly, intraperitoneally or intraventricularly. In a preferred embodiment, the composition is administered intravenously, intraarterially or intraventricularly. More preferably, the composition is administered intravenously.

Any amount of cells can be administered to a patient as long as the dosage comprises a therapeutically effective amount. In a preferred embodiment, at least 1x10⁵ cells per kilogram (kg) of the patient's body weight are administered. Preferably, at least 1x10⁵, 2x10⁵, 3x10⁵, 4x10⁵, 5x10⁵, 6x10⁵, 7x10⁵, 8x10⁵ or 9x10⁵ cells are administered per kg of the patient's body weight. More preferably, approximately 1x10⁶ cells are administered per kg of the patient's body weight. The term "approximately" refers to a variability of ± 10%.

In a preferred embodiment, the composition is used in a combination therapy with one or more therapeutic agents selected from the group consisting of angiotensin converting enzyme inhibitors, angiotensin II receptor blockers, beta blockers, diuretics, antagonists. aldosterone, inotropic and digoxin and / or in combination with a surgical intervention or medical device selected from the group consisting of coronary bypass surgery, repair or replacement of heart valve, implantable cardioverter defibrillator, cardiac resynchronization therapy, bi-ventricular stimulation, pumps Heart and heart transplants.

Examples of said therapeutic agents are mentioned in example 1.

In one embodiment, the composition of the invention is administered only once. It is preferably administered 2 or more times.

### Results of the treatment

To evaluate the efficacy of the therapy, a change in the left ventricular ejection fraction (LVEF) in echocardiography is sought. In addition, an absolute 5% increase in LVEF evaluated by echocardiography was considered as a clinically significant response to therapy.

Next, the follow-up of the 3 patients treated with the cellular composition described in this document is shown in Figure 22.

As can be seen, the patients improved their left ventricular ejection fraction at 3, 6 and 12 months. The absolute increase is greater than 14%, indicating a significant clinical response. Therefore, it is demonstrated that the cellular compositions developed by the methodology described in this document benefit patients with heart failure with reduced ejection fraction.

It should be noted that in the patients mentioned no immediate adverse events were observed after the intravenous infusion of the composition of the invention; Incidence of global death, major cardiovascular events (defined by the combined outcome of cardiovascular deaths, hospital admission due to decompensated heart failure (HF), non-fatal myocardium heart attack) and other adverse events including heart attacks, sustained ventricular arrhythmias and malignant incidents.

### Example 7. Use of Mesenchymal Subpopulation for patients suffering from respiratory injuries

This example consists in the use of a cellular composition selected by the method of selection of the invention to treat patients with respiratory lesions, such as a lung injury, an acute lung injury or acute respiratory distress syndrome. Specifically, this example consists in the treatment of a patient presenting with acute respiratory distress syndrome.

### Inclusion criteria

Candidates for the treatment of the invention are selected from patients suffering from respiratory injury. Especially patients who have any type of acute lung injury, such as acute respiratory distress syndrome of any etiology. Patients who have an oxygen blood pressure ratio versus the inspired oxygen fraction PAO2 / FIO2 (known as PAFI or Kirby Index) of less than 200 are also considered candidates for the treatment of the invention.

For this example, 1 patient was treated with the developed cell composition of batch CU-668-K that complies with the method of selection of the invention.

### Administration

Any of the compositions approved by the selection criteria "Characterization of MSC subpopulations" described herein, can be administered to any of the patients described, by any of the means described below.

The composition can be administered through any known method. For example, the composition can be administered intravenously, intralesionally, intrapulmonally, intravascularly, intraarterially, subcutaneously, intracerebrally, intramuscularly, intraperitoneally or intraventricularly. In a preferred embodiment, the composition is administered intralesionally, subcutaneously or intraarticularly. Preferably, the composition is administered intra-articular.

Any amount of cells can be administered to a patient as long as the dosage comprises a therapeutically effective amount. In a preferred embodiment, at least 1x10⁴ cells per kilogram of the patient are administered. Preferably, at least 5 x 10⁴, 1 x 10⁵ or 5 x 10⁵ cells are administered per kilogram of the patient. More preferably, at least about 8x10⁵ cells are administered per kilogram of the patient. The term "approximately" refers to a variability of ± 10%.

Most preferably, a total of 1x10⁶ cells per kilogram of the patient will be injected intravenously, in a time of at least 15 min.

In one embodiment, the composition of the invention is administered only once. It is preferably administered 2 or more times.

### Results of the treatment

To evaluate the efficacy of the therapy, we seek to observe an improvement in pulmonary inflammation, characterized by alveolar condensation and by the visualization of the frosted glass pattern (greyish) of the lungs on a CT scan of the chest.

Figure 23 shows the result for the treatment of the patient suffering from acute respiratory distress syndrome with the cell composition selected by the methodology described in this document. As can be seen, at 13 days after receiving the treatment, a decrease in acute pulmonary infiltrates is seen. Specifically, in computed tomography it is possible to visualize a significant decrease in the areas of alveolar condensation and frosted glass pattern, with persistence of areas of pulmonary fibrosis secondary to chronic damage induced by acute pulmonary injury. This tomographic improvement is associated with an improvement in gas exchange and pulmonary compliance.

### Therefore, the cellular composition selected by the method described herein is effective for treating a lung injury, an acute lung injury, such as acute respiratory distress syndrome.

### Example 8. Use of Mesenchymal Subpopulation to treat skin wounds

This example consists in the use of a cellular composition selected by the selection method described herein to treat skin wounds. For this, a mouse model with a cleavage wound based on the "mouse excisional wound splinting model" model [35] was used to be treated with the selected cell compositions. In this example an intradermal injection of the selected cellular compositions was used, in which 7 animals treated with the compositions were compared versus 9 animals treated with a saline solution.

For this example, 2 cell compositions selected using the methodology described herein were used, the lots used were 108-6 and 745-3.

### Administration

Any of the compositions approved by the selection criteria "Characterization of MSC subpopulations" described herein, can be administered to any of the patients described, by any of the means described below.

The composition can be administered through any known method. For example, the composition can be administered intravenously, intradermally, intralesionally, intraarticularly, intravascularly, intraarterially, subcutaneously, intracerebrally, intramuscularly, intraperitoneally or intraventricularly. In a preferred embodiment, the composition is administered intralesionally, subcutaneously or intraarticularly. Preferably, the composition is administered intradermally.

Any amount of cells can be administered to a patient as long as the dosage comprises a therapeutically effective amount. In a preferred embodiment, at least 1x10⁵ cells are administered per square centimeter of wound. Preferably, at least 10 x 10⁵, 20 x 10⁵ or 25 x 10⁵ cells are administered per square centimeter of wound. More preferably, at least about 3x10⁶ cells are administered per square centimeter of wound. The term "approximately" refers to a variability of ± 10%.

Most preferably, a total of 3.5x10⁶ cells per square centimeter of wound will be injected intradermally, either as a patch or scalfold.

In one embodiment, the composition of the invention is administered only once. It is preferably administered 2 or more times.

### Results of the treatment

To evaluate the effectiveness of the therapy, an improvement in tissue regeneration is sought, either in the closure of the wound over time or in the formation of vessels in the tissue.

The results for the treatment of cellular composition described in this document are shown in Figure 24. As can be seen, the cellular compositions show a better wound closure during the fourteen days (Figure 24A). Specifically, the cell composition has a 14-day closing percentage of 94.67 +/- 1,136 (SEM, standard error of the average), while the saline solution has a closing percentage of only 84.14 +/- 3,322 (SEM), this difference is statistically significant (Figure 24B). For angiogenesis, the cellular composition has a much higher percentage of vessel formation in the wound compared to saline solution (Figure 24C), specifically the cellular composition has a vessel formation of 68.10 +/-6.247 (SEM) versus the salt composition with a 49.2 +/- 5,755 (SEM), this difference is statistically significant.

### Therefore, cell compositions selected by the method described herein benefit the regeneration of skin wounds.

### References

[1] Dominici, M., Le Blanc, K., et al. (2006). Minimal criteria for defining multipotent mesenchymal stromal cells. The International Society for Cellular Therapy position statement. Cytotherapy, 8(4), 315-317. https://doi.org/10.1080/14653240600855905.
[2] Larijani, B., Esfahani, E. N., et al. (2012). Stem cell therapy in treatment of different diseases. Acta Med Iran, 50, 79-96. https://doi.org/20202 [pii].
[3] El-Badawy, A., & El-Badri, N. (2016). Clinical efficacy of stem cell therapy for diabetes mellitus: A meta- analysis. PLoS ONE, 11(4), 1-16. https://doi.org/10.1371/journal.pone.0151938.
[4] Lunde K, Solheim S, Aakhus S, Arnesen H, , et al. Intracoronary injection of mononuclear bone marrow cells in acute myocardial infarction, New Eng J Med. 2006 Sep 21;355(12):1199-209.
[5] Perin EC, Silva GV, et al. A randomized study of transendocardial injection of autologous bone marrow mononuclear cells and cell function analysis in ischemic heart failure (FOCUS-HF). Am Heart J. 2011 Jun;161(6):1078-87.
[6] Hare, J. M., Traverse, J. H., et al. (2009). A Randomized, Double-Blind, Placebo-Controlled, Dose-Escalation Study of Intravenous Adult Human Mesenchymal Stem Cells (Prochymal) After Acute Myocardial Infarction. Journal of the American College of Cardiology, 54(24), 2277-2286.
[8] Strauer BE, Yousef M, Schannwell CM. The acute and long-term effects of intracoronary stem cell transplantation in 191 patients with chronic heart failure: the STAR-heart study. Eur J Heart Fail. 2010 Jul;12(7):721-9.
[9] Seth S, Bhargava B, Narang R, Ray R, Mohanty S, Gulati G, et al.; AIIMS Stem Cell Study Group. The ABCD (Autologous Bone Marrow Cells in Dilated Cardiomyopathy) trial a long-term follow-up study. J Am Coll Cardiol. 2010 Apr 13;55(15):1643-4.
[10] Barry F, Murphy M. Mesenchymal stem cells in joint disease and repair. Nat Rev Rheumatol 2013;9:584- 594.
[11] Varma HS, Dadarya B, Vidyarthi A. The new avenues in the management of osteoarthritis of knee-stem cells. J Indian Med Assoc 2010;108:583-585.
[12] Peng X, Xiaoju W, Qiang L, Xueping L. Efficacy of mesenchymal stem cells injection for the management of knee osteoarthritis: a systematic review and meta-analysis. International Orthopaedics 2015; doi 10.1007/s00264-015-2785-8.
[13] Vangsness CT, Farr J, Boyd J, Dellaero DT, Mills CR, LeRoux-Williams M. Adult human mesenchymal stem cells delivered via intra-articular injection to the knee following partial medial meniscectomy: a randomized, double-blind, controlled study. J Bone Joint Surg Am 2014;96(2):90-8.
[14] Ishimine H, et. Al. N-Cadherin is a prospective cell surface marker of human mesenchymal stem cells that have high ability for cardiomyocyte differentiation. Biochem Biophys Res Commun. 2013 Sep 6;438(4):753-9. doi: 10.1016/j.bbrc.2013.07.081. Epub 2013 Jul 27.
[15] Di Benedettoa A., et al. Osteogenic differentiation of mesenchymal stem cells from dental bud: Role of integrins and cadherins. Stem Cell Research. Nov 2015. Volume 15, Issue 3, Pages 618-628
[16] Xu, Liangliang & Meng, Fanbiao et al. (2012). N-cadherin regulates osteogenesis and migration of bone marrow-derived mesenchymal stem cells. Molecular biology reports. 40. 10.1007/s11033-012-2334-0.
[17] Ponta, H., Sherman, L., & Herrlich, P. A. (2003). CD44: From adhesion molecules to signalling regulators. Nat Rev Mol Cell Biol, 4(1), 33-45. Retrieved from http://dx.doi.org/10.1038/nrm1004.
[18] Chung C, Burdick JA. Influence of 3D Hyaluronic Acid Microenvironments on Mesenchymal Stem Cell Chondrogenesis. Tissue engineering Part A. 2009;15(2):243-254. doi:10.1089/ten.tea.2008.0067.
[19] Sharaf-Eldin WE, Abu-Shahba N, Mahmoud M, El-Badri N. The Modulatory Effects of Mesenchymal Stem Cells on Osteoclastogenesis. Stem Cells International. 2016; 2016:1908365. doi:10.1155/2016/1908365.
[20] Valluru M, Staton CA, Reed MWR, Brown NJ. Transforming Growth Factor-β and Endoglin Signaling Orchestrate Wound Healing. Frontiers in Physiology. 2011;2:89. doi:10.3389/fphys.2011.00089.
[21] Neuss, S., Becher, E., Wöltje, M., Tietze, L. and Jahnen-Dechent, W. (2004), Functional Expression of HGF and HGF Receptor/c-met in Adult Human Mesenchymal Stem Cells Suggests a Role in Cell Mobilization, Tissue Repair, and Wound Healing. STEM CELLS, 22: 405-414. doi:10.1634/stemcells.22-3-405.
[22] Miyazawa, K. (2010), Hepatocyte growth factor activator (HGFA): a serine protease that links tissue injury to activation of hepatocyte growth factor. FEBS Journal, 277: 2208-2214. doi:10.1111/j.1742- 4658.2010.07637.
[23] Zhao, L., Liu, X., Zhang, Y., Liang, X., Ding, Y., Xu, Y., ... Zhang, F. (2016). Enhanced cell survival and paracrine effects of mesenchymal stem cells overexpressing hepatocyte growth factor promote cardioprotection in myocardial infarction. Experimental Cell Research, 344(1), 30-39. https://doi.org/10.1016/j.yexcr.2016.03.024.
[24] Wang, J., Sun, B., Tian, L., He, X., Gao, Q., Wu, T., ... Mo, X. (2017). Evaluation of the potential of rhTGF- β3 encapsulated P(LLA-CL)/collagen nanofibers for tracheal cartilage regeneration using mesenchymal stems cells derived from Wharton's jelly of human umbilical cord. Materials Science and Engineering C, 70, 637-645. https://doi.org/10.1016/j.msec.2016.09.044
[25] Wu, Y., Peng, Y., Feng, C., Yuan, X., Li, H., Wang, Y., ... Fu, X. (2015). Mesenchymal Stem Cells Suppress Fibroblast Proliferation and Reduce Skin Fibrosis Through a TGF-β3-Dependent Activation. The International Journal of Lower Extremity Wounds, 14(1), 50-62. https://doi.org/10.1177/1534734614568373.
[26] Wang, Y., Zhao, X., Huojia, M., Xu, H., & Zhuang, Y. (2016). Transforming growth factor-β3 promotes facial nerve injury repair in rabbits. Experimental and Therapeutic Medicine, 11(3), 703-708. https://doi.org/10.3892/etm.2016.2972
[27] Morrison, T. J., Jackson, M. V et al.. (2017). Mesenchymal Stromal Cells Modulate Macrophages in Clinically Relevant Lung Injury Models by Extracellular Vesicle Mitochondrial Transfer. American Journal of Respiratory and Critical Care Medicine, 196(10), 1275-1286. http://doi.org/10.1164/rccm.201701-01700C
[28] Bocelli-Tyndall C, Trella E, Frachet A, et al. FGF2 induces RANKL gene expression as well as IL1β regulated MHC class II in human bone marrow-derived mesenchymal progenitor stromal cells. Annals of the Rheumatic Diseases 2015;74:260-266. https://ard.bmj.com/content/74/1/260
[29] Wang, Y. Y.; Li, X. Z.; Wang, L. B. Therapeutic implications of mesenchymal stem cells in acute lung injury/acute respiratory distress syndrome. Stem Cell Res. Ther. 4(3): 45; 2013. https://doi.org/10.1186/scrt193
[30] Fonsatti, E., & Maio, M. (2004). Highlights on endoglin (CD105): from basic findings towards clinical applications in human cancer. Journal of Translational Medicine, 2, 18. http://doi.org/10.1186/1479-5876-2- 18
[31] Voelkel, N. F., Douglas, I. S., & Nicolls, M. (2007). Angiogenesis in Chronic Lung Disease. Chest, 131(3), 874-879. http://doi.org/10.1378/chest.06-2453
[32] Morrison, T., McAuley, D., & Krasnodembskaya, A. (2015). Mesenchymal stromal cells for treatment of the acute respiratory distress syndrome: The beginning of the story. Journal of the Intensive Care Society, 16(4), 320-329. http://doi.org/10.1177/1751143715586420
[33] Skog, M. S., Nystedt, J., et al. (2016). Expression of neural cell adhesion molecule and polysialic acid in human bone marrow-derived mesenchymal stromal cells. Stem Cell Research & Therapy, 7, 113. http://doi.org/10.1186/s13287-016-0373-5
[34] Lee, E. J., Choi, et al.. (2012). N-cadherin Determines Individual Variations in the Therapeutic Efficacy of Human Umbilical Cord Blood-derived Mesenchymal Stem Cells in a Rat Model of Myocardial Infarction. Molecular Therapy, 20(1), 155-167. http://doi.org/10.1038/mt.2011.202
[35] Ahn, S. Y., Park, W. S., et al. (2018). Vascular endothelial growth factor mediates the therapeutic efficacy of mesenchymal stem cell-derived extracellular vesicles against neonatal hyperoxic lung injury. Experimental & Molecular Medicine, 50(4), 26. https://doi.org/10.1038/s12276-018-0055-8
[36] Senbanjo, L. T., & Chellaiah, M. A. (2017). CD44: A Multifunctional Cell Surface Adhesion Receptor Is a Regulator of Progression and Metastasis of Cancer Cells. Frontiers in Cell and Developmental Biology, 5, 18. http://doi.org/10.3389/fcell.2017.00018
[37] Wang W, Li P, Li W, Jiang J, Cui Y, et al. (2017) Osteopontin activates mesenchymal stem cells to repair skin wound. PLOS ONE 12(9): e0185346.https://doi.org/10.1371/journal.pone.0185346
[38] Liu, L., Luo, Q., Sun, J., Wang, A., Shi, Y., Ju, Y., Song, G. (2017). Decreased nuclear stiffness via FAK-ERK1/2 signaling is necessary for osteopontin-promoted migration of bone marrow-derived mesenchymal stem cells. Experimental Cell Research, 355(2), 172-181. https://doi.org/https://doi.org/10.1016/j.yexcr.2017.04.004
[39] Schaffer, M., Bongartz, M., Hoffmann, W., & Viebahn, R. (2007). MHC-Class-II-Deficiency Impairs Wound Healing. Journal of Surgical Research, 138(1), 100-105. https://doi.org/10.1016/j.jss.2006.05.029

## Claims

1. Method of obtaining a composition that contains a specific population of umbilical cord mesenchymal cells CHARACTERIZED because it comprises the following stages:
a) cultivating and expanding umbilical cord mesenchymal cells,
b) select them for the presence of at least 3 of the markers N-cadherin, CD44, RANKL, CD105 and CD56.

2. Method according to claim 1 CHARACTERIZED because optionally after step b) a selection is made by production of hepatocyte growth factor (HGF), selecting the cultures that secrete more than 1000 pg / mL of HGF in the culture medium.

3. Method according to claim 2, CHARACTERIZED because the cultures that secrete more than 1500 pg / mL of HGF in the culture medium are selected.

4. Method according to claim 3 CHARACTERIZED because the cultures that secrete more than 2000 pg / mL of HGF in the culture medium are selected.

5. Method according to claim 4, CHARACTERIZED because the cultures that secrete more than 2500 pg / mL of HGF in the culture medium are selected.

6. Method according to claim 1, **CHARACTERIZED in that** the umbilical cord mesenchymal cells are optionally specifically obtained from the connective tissue cells that surround the arteries and the umbilical vein and that are adjacent to the internal face of the subaminiotic layer.

7. Method according to claim 1 CHARACTERIZED because optionally after step b) an optional selection is made by expression of TGF-β3, selecting the cultures that express the highest levels of relative expression compared to the samples evaluated.

8. Method according to claim 6, CHARACTERIZED because in the umbilical cord the amniotic and subaminiotic layers are first removed, second, 3 longitudinal and parallel cuts are made through the arteries and the umbilical vein in order to expose the vein and the arteries, and remove them from the tissue.

9. Method according to claim 8 **CHARACTERIZED in that** the cuts obtained clean from arteries and veins are placed in plates with culture medium so that the connective tissue that was adjacent to the vein and arteries is in direct contact with the plate of culture.

10. Method according to claim 1 CHARACTERIZED because the cells in step a) are cultured for up to 10 days.

11. Method according to claim 10, CHARACTERIZED because the cells in stage a) are cultured for up to 8 days.

12. Method according to claim 10 or 11, CHARACTERIZED because the cultured cells are selected by cytometry, according to their markers, selecting as mesenchymal cells those that express more than 95% CD105, CD73 and CD90, and which at the same time do not express, that is, they express less than 2% CD45, CD34, CD14 and HLA-DR.

13. Method according to claim 11 CHARACTERIZED because the selected mesenchymal cells expand and separate according to the provisions of step b).

14. Method according to claim 1 CHARACTERIZED because in step c) the cells are selected because they express N-cadherin and at least 2 of the markers, CD105, CD44, RANKL or CD56.

15. Method according to claim 14 CHARACTERIZED because in step b) the cells are selected because they express N-cadherin, RANKL and at least 1 of the markers, CD105, CD44, or CD56.

16. Method according to claim 15 CHARACTERIZED because in step b) the cells are selected because they express N-cadherin, RANKL and at least 2 of the markers, CD105, CD44, or CD56.

17. Method according to claim 16, CHARACTERIZED because in step b) the cells are selected because they express N-cadherin, RANKL, CD105, CD44, and CD56.

18. Composition containing a specific population of umbilical cord mesenchymal cells CHARACTERIZED because it comprises mesenchymal cells that express N-cadherin and at least 2 of the markers, CD105, CD44, RANKL or CD56, and which is obtained by the method of claims 1 to 17.

19. Use of the composition containing cells from a specific population of umbilical cord mesenchymal cells obtained according to the method of claims 1 to 17 or as defined in claim 18 CHARACTERIZED because it serves to prepare a medication useful for cell therapies.

20. Use according to claim 19 CHARACTERIZED because it serves to prepare a useful medication to treat cardiac ejection flow problems.

21. Use according to claim 19 CHARACTERIZED because it serves to prepare a useful medication to treat cardiac ejection flow problems that is administered intravenously.

22. Use according to claim 21 CHARACTERIZED because it serves to prepare a useful medication to treat heart failure.

23. Use according to claim 19 CHARACTERIZED because it serves to prepare a useful medication to treat pain and improve functionality associated with joint wear problems.

24. Use according to claim 23 CHARACTERIZED because it serves to prepare a useful medication to treat wear and tear in the interaction between the femur, tibia and patella.

25. Use according to claim 23 CHARACTERIZED because it serves to prepare a useful medication to treat wear between the head of the femur with the acetabulum.

26. Use according to claim 23 CHARACTERIZED because it serves to prepare a useful medication to treat wear between the talus, tibia and fibula.

27. Use according to claim 23 CHARACTERIZED because it serves to prepare a useful medication to treat wear between the radius, ulna and carpal / carpal bones.

28. Use according to claim 23 CHARACTERIZED because it serves to prepare a useful medication to treat wear and tear on knee joints.

29. Use according to claim 23 CHARACTERIZED because it serves to prepare a useful medication to treat wear and tear on ankle, hip, knee, wrist, shoulder, finger, elbow, neck and spinal joints.

30. Use according to claim 19 CHARACTERIZED because it serves to prepare a useful medication to treat respiratory problems.

31. Use according to claim 30 CHARACTERIZED because it serves to prepare a useful medication to treat lung injuries.

32. Use according to claim 31 CHARACTERIZED because it serves to prepare a useful medication to treat acute lung injuries.

33. Use according to claim 32 CHARACTERIZED because it serves to prepare a useful medication to treat acute respiratory distress syndrome.

34. Use according to claim 19 CHARACTERIZED because it serves to prepare a useful medication to treat skin lesions.

35. Use according to claim 34 CHARACTERIZED because it serves to prepare a useful medication to treat chronic wounds.

36. Use according to claim 34 CHARACTERIZED because it serves to prepare a medication useful to treat skin ulcers.
